(19) Europäisches Patentamt / European Patent Office / Office européen des brevets

(11) **EP 4 419 717 B1**

(12) **EUROPEAN PATENT SPECIFICATION**

(45) Date of publication and mention
of the grant of the patent:
**03.12.2025 Bulletin 2025/49**

(21) Application number: **22808666.6**

(22) Date of filing: **19.10.2022**

(51) International Patent Classification (IPC):
***C12Q 1/6886*** (2018.01)

(52) Cooperative Patent Classification (CPC):
**C12Q 1/6886;** C12Q 2600/154

(86) International application number:
**PCT/EP2022/079056**

(87) International publication number:
**WO 2023/066972 (27.04.2023 Gazette 2023/17)**

(54) **DNA METHYLATION SIGNATURE FOR DIAGNOSING HEPATOCELLULAR CARCINOMA**

DNA-METHYLIERUNGSSIGNATUR ZUR DIAGNOSE VON HEPATOZELLULÄREM KARZINOM

SIGNATURE DE MÉTHYLATION D'ADN POUR DIAGNOSTIQUER LE CARCINOME HÉPATOCELLULAIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **19.10.2021 EP 21306459**

(43) Date of publication of application:
**28.08.2024 Bulletin 2024/35**

(73) Proprietors:
• **INSERM (Institut National de la Santé et de la Recherche Médicale)**
**75013 Paris (FR)**
• **Université de Lorraine**
**54000 Nancy (FR)**

• **Centre Hospitalier Régional Universitaire de Nancy**
**54000 Nancy (FR)**

(72) Inventor: **OUSSALAH, Abderrahim**
**54850 Messein (FR)**

(74) Representative: **Plasseraud IP**
**104 Rue de Richelieu**
**CS92104**
**75080 Paris Cedex 02 (FR)**

(56) References cited:
WO-A1-2016/101258    WO-A1-2021/146570
CN-A- 110 904 231    US-A1- 2020 115 761
US-A1- 2020 255 904    US-A1- 2021 171 617

**Description**

**TECHNOLOGY FIELD OF THE INVENTION**

The inventors report in the present application the results of applying an innovative analytical approach on three independent EWASs for deriving and validating an epigenome score that exhibited a high diagnostic accuracy for detecting hepatocellular carcinoma (HCC). The present invention relates to the use of a DNA methylation signature for diagnosing hepatocellular carcinoma in a subject.

**BACKGROUND OF THE INVENTION**

Hepatocellular carcinoma (HCC) is the most common primary malignant tumor of the liver (El-Serag HB. Hepatocellular carcinoma. N Engl J Med 2011;365:1118-1127; Bruix J, Reig M, Sherman M. Gastroenterology 2016;150:835-853; Oussalah A, et al. Oncotarget 2016). It is the fifth most common cancer in men and the seventh in women and ranks second in annual cancer mortality rates worldwide, with liver cancer diagnosed in >700,000 people annually (El-Serag HB. Hepatocellular carcinoma. N Engl J Med 2011;365:1118-1127; Bruix J, Reig M, Sherman M. Gastroenterology 2016;150:835-853, World Health Organization. Cancer Fact sheet. 2017. http://www.who.int/media centre/factsheets/fs297/en/ (accessed 15 December 2017)). Major risk factors for HCC include cirrhosis, hepatitis B (HBV) or C virus (HCV) infections, alcoholic liver disease, and non-alcoholic fatty liver disease. The 5-year cumulative risk for HCC in patients with cirrhosis ranges between 5 and 30%, depending on the underlying condition (El-Serag HB. Hepatocellular carcinoma. N Engl J Med 2011;365:1118-1127).

Epigenetic alterations are a common hallmark of human cancer (Moran S, et al. Nat Rev Clin Oncol 2017, Hao X, et al. Proc Natl Acad Sci U S A 2017;114:7414-7419). In this setting, the advent of genome-wide methods for assessing the epigenome landscape, notably the DNA methylome, has revolutionized the understanding of the epigenome architecture of cancers (Michels KB, et al. Nat Methods 2013;10:949-955). Epigenome-wide association studies (EWAS) use microarrays to analyze the methylation of CpG dinucleotides at the genome-wide level. The ability of EWAS to translate into biologically meaningful results relies on the detection of epigenomic signatures with a high level of statistical certainty. However, EWAS may be prone to bias, potentially leading to spurious associations, notably in case series with small sample sizes (Michels KB, et al. Nat Methods 2013;10:949-955; van Iterson M, van Zwet EW, Consortium B, Heijmans BT. Genome Biol 2017;18:19). WO 2021/146570 discloses a method of diagnosing and treating hepatocellular carcinoma (HCC) in a patient, the method comprising detecting methylation at one or more CpG of one or more genes selected from a group comprising RUNX3 and SEPT9.

**SUMMARY OF THE INVENTION**

The inventors developed a smoothing method for calculating and visualizing data from EWASs and demonstrated its utility for identifying highly accurate epigenomic signatures in DNA methylation analyses. The application of the smoothing method to several settings of EWASs confirmed its usefulness for deciphering the most informative epigenomic signatures with a high degree of certainty while controlling the risk of spurious findings outside the significant loci at a genome-wide level. The inventors report in the present application the results of applying this innovative analytical approach on three independent EWASs for deriving and validating an epigenome score that exhibited a high diagnostic accuracy for detecting hepatocellular carcinoma (HCC).

The present invention relates to a method for diagnosing hepatocellular carcinoma (HCC) in a subject comprising the step of determining the methylation level of at least one CpG site within each of CpG islands listed in Table 1 in DNA sample obtained from said subject, wherein a higher methylation level of said CpG sites as compared to a control value is indicative that the subject has a hepatocellular carcinoma, preferably wherein said CpG sites are selected from the group consisting of CpG sites listed in Table 1.

In another aspect, the present invention relates to a kit for predicting hepatocellular carcinoma disease in a subject which comprises a set of probes which each hybridizes with a nucleic acid sequence comprising at least one CpG site within each CpG island listed in Table 1, preferably CpG sites selected from the group consisting of CpG sites listed in Table 1, preferably which further comprises a set of probes which each hybridizes with a nucleic acid sequence comprising at least one CpG site within each CpG islands listed in Table 1, wherein cytosines are replaced by uracils, preferably wherein said CpG sites are selected from the group consisting of CpG sites listed in Table 1.

**FIGURE LEGENDS**

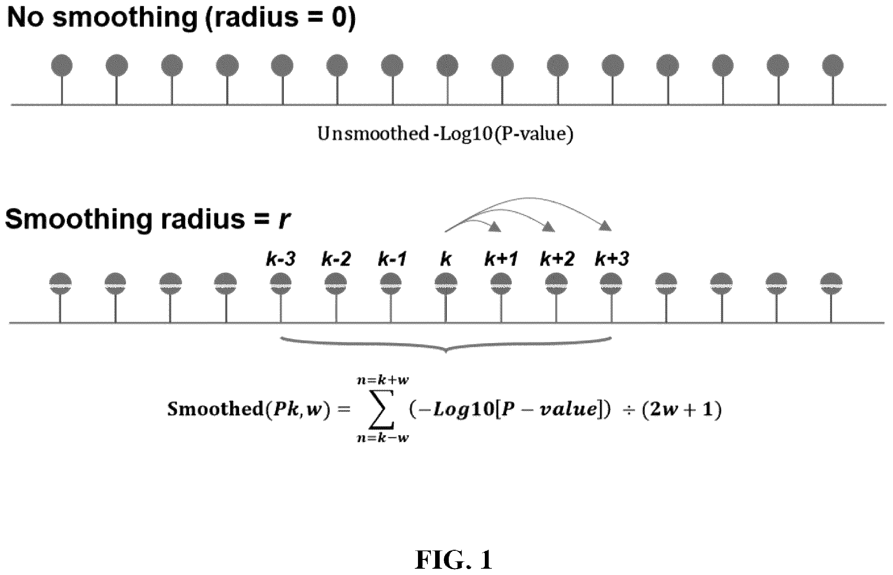

**Figure 1. Conceptual representation of the smoothing method for calculating and visualizing data from epigenome-wide association studies.** The smoothing method relies on the DNA co-methylation pattern, which is the correlation between adjacent CpG sites. The smoothing method consists of the conversion of the nominal -Log10($P$-value) obtained for each CpG probe at the genome-wide level to smoothed P-values (Smoothed[$P_{k,w}$]) using a pre-specified window width value ($w$). A detailed description of the smoothing method and the corresponding statistical formula are reported in Online Methods.

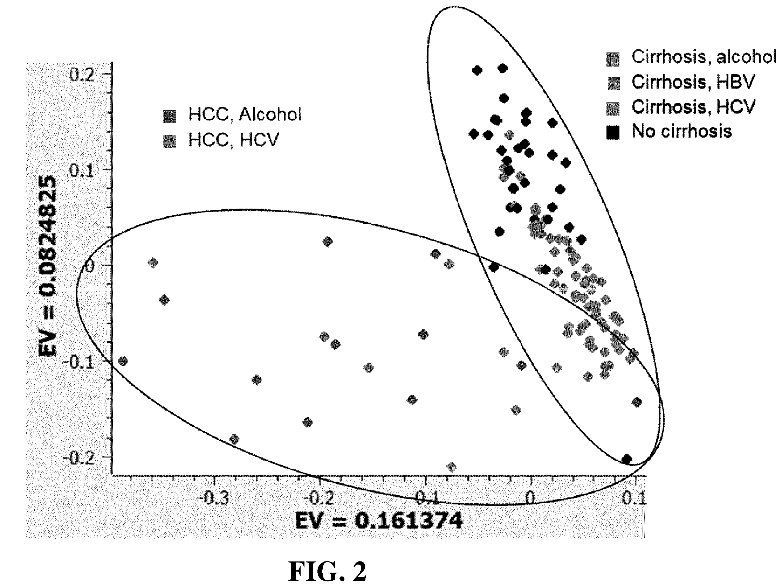

**Figure 2.** Two-dimensional plot using two top eigenvectors derived from the primary component analysis on the genome-wide methylome landscape of the studied HCC, non-HCC cirrhotic, and normal liver samples.

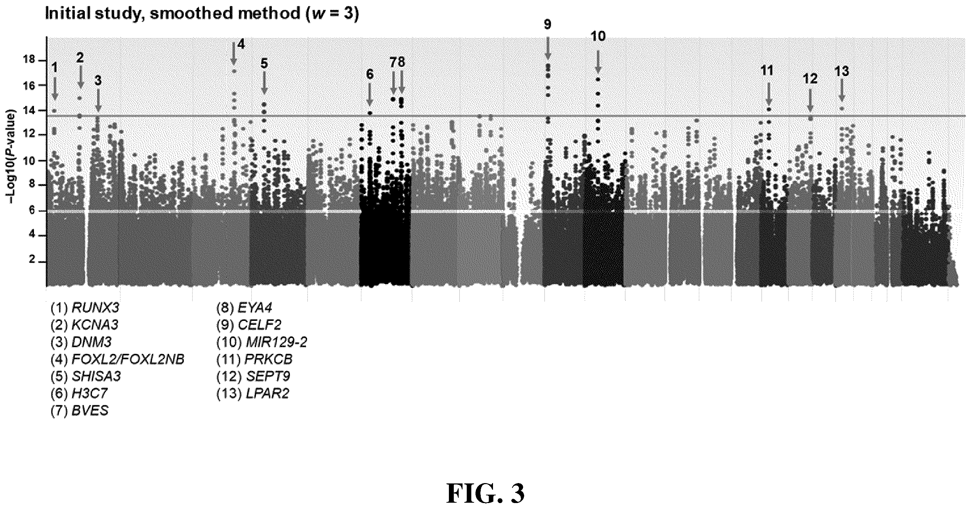

**Figure 3.** Epi-Manhattan plot reporting the results of the epigenome-wide association study for the hepatocellular phenotype in the initial study. The arrows indicate the top significant loci and the numbering indicates the genes corresponding to the 13 top loci in the genomic order.

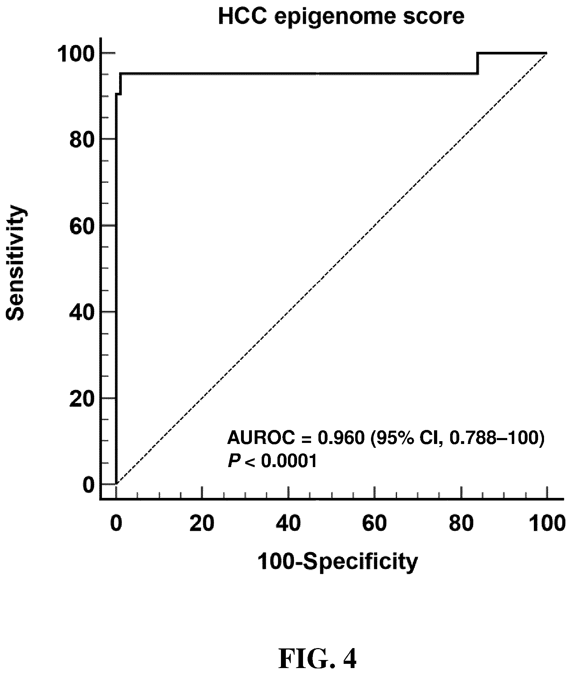

**Figure 4.** ROC curve for the diagnostic accuracy of the HCC Epigenome Score for the detection of the HCC phenotype.

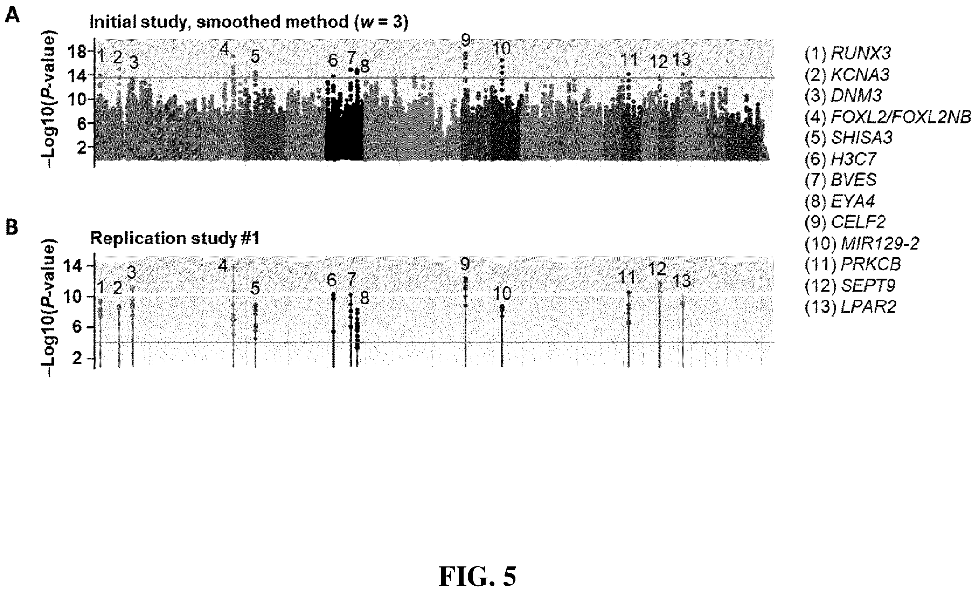

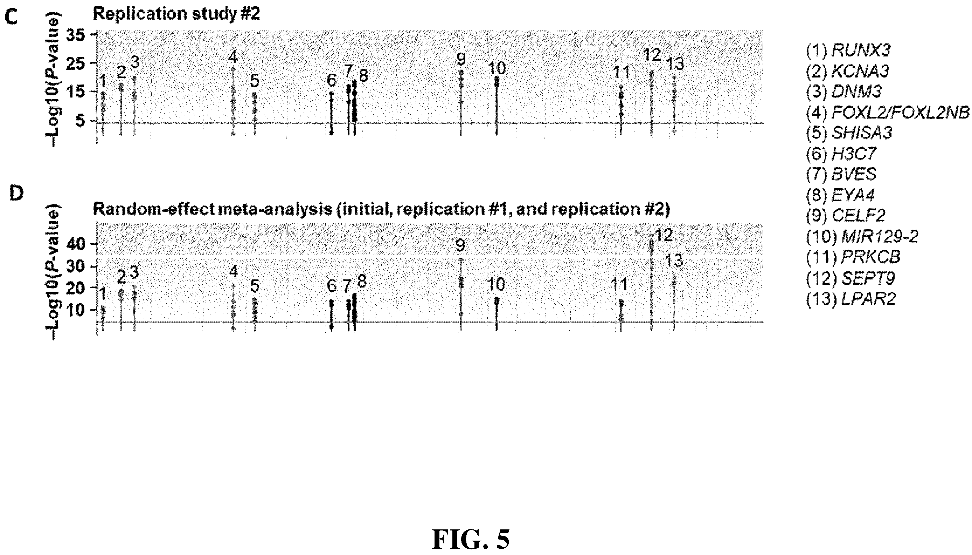

**Figure 5.** Epi-Manhattan plot reporting the results of the epigenome-wide association study for the hepatocellular phenotype in the initial study (A), and for the epigenome association studies on the 105 top CpG probes in replication studies #1 (B) and #2 (C). Panel D reports the epi-Manhattan plot of the random-effect meta-analysis that pooled epigenome association studies on the 105 top CpG probes from the initial and the replication #1 and #2 datasets.

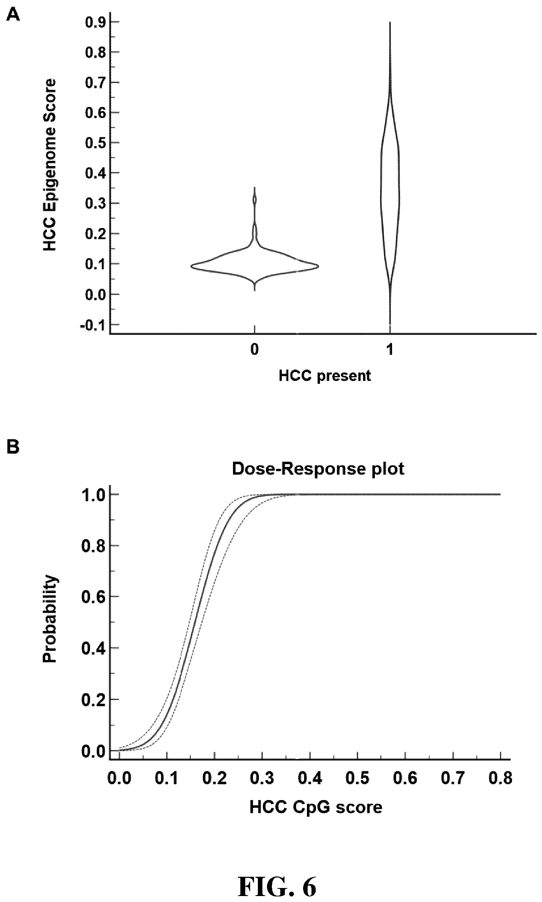

**Figure 6. (A)** Violin plot reporting the density distribution of the HCC Epigenome Score in the 353 HCC samples and the 233 non-HCC; (B) Probit sigmoid dose-response curve showing the HCC Epigenome Score as the dose variable and the HCC status as the response variable. The central line shows the probability and corresponding dose. The dashed curves represent the 95% confidence interval for the respective dose. The dose and 95% confidence interval corresponding with a particular probability are taken from a horizontal line at that probability level.

## DETAILED DESCRIPTION OF THE INVENTION

### Definitions

As used herein, the term "nucleic acid sequence" refers to a single- or double-stranded nucleic acid. Said nucleic acid sequence can be DNA or RNA. In preferred embodiments, the "nucleic acid sequence" is a double-stranded DNA.

The term "DNA methylation profile" or "methylation profile" refers to a pattern of methylation level of cytosine of a set of CpG sites of determined genomic DNA positions in DNA sample that generally occurs as a result of a biological process, e.g. pathological or physiological state.

As used herein, the term "CpG site" or "CpG probe" refers to the position in DNA sequence where a cytosine and guanine are adjacent and the 3'oxygen of the cytosine is covalently attached to the 5'Phosphate of the guanine. Cytosines in CpG dinucleotides can be methylated to form 5-methylcytosine.

As used herein, the term "methylation state" or "methylation level" as used herein describes the state of methylation of a CpG site, thus refers to the presence or absence of 5-methylcytosine at one cpG site within DNA. When none of the DNA of an individual is methylated at one given CpG site, the position is 0% methylated. When all the DNA of the individual is methylated at that given CpG site, the position is 100% methylated. When only a portion, e.g., 50%, 75%, or 80% of the DNA of the individual is methylated at said CpG site, then the CpG position is said to be 50%, 75%, 80% methylated, respectively.

The term "methylation level" reflects any relative or absolute amount of methylation of a CpG position. The terms "methylation" and "hypermethylation" are used herein interchangeably. When used in reference to a CpG position, they refer to the methylation status corresponding to an increased or decreased presence of 5-methylcytosine at a CpG site within the DNA sequence of a biological sample obtained from a population (e.g. patient) relative to the amount of 5-methylcytosine found at the CpG site within the same DNA position of a biological sample obtained from a control value (e.g. healthy individual).

In a particular embodiment, said methylation state at the CpG site is a beta-value that corresponds to the ratio of methylated cytosine and the sum of methylated and unmethylated cytosines at the CpG site.

The term "CpG-rich region" refers herein to a genomic DNA region of at least 200 bp that has more than 50% CpG content. In particular embodiment, said GpG rich region is CpG island.

The term "subject" refers to both human and non-human animals. As used herein, the term "subject" denotes a mammal, such as a rodent, a feline, a canine, and a primate. Preferably, a subject according to the invention is a human.

The term "biological sample" or "sample" is generally obtained from a subject or from a population of subjects. A sample may be any biological tissue or fluid with which the methylation status of CpG sites of the present disclosure may be assayed. Frequently, a sample will be a "clinical sample" (i.e., a sample obtained or derived from a patient to be tested). The sample may also be an archival sample with a known diagnosis, treatment, and/or outcome history. Examples of biological samples suitable for use in the practice of the present disclosure include, but are not limited to, bodily fluids, e.g., blood samples (e.g., blood smears), and cerebrospinal fluid, brain tissue samples or bone marrow tissue samples such as tissue or fine needle biopsy samples. Biological samples may also include sections of tissues, such as frozen sections taken for histological purposes. The term "biological sample" also encompasses any material derived by processing a biological sample. Derived materials include, but are not limited to, cells (or their progeny) isolated from the sample, as well as nucleic acid molecules (DNA and/or RNA) extracted from the sample. The biological sample may be circulating free DNA (cfDNA), which corresponds to degraded DNA fragments released to the blood plasma. cfDNA can be derived from healthy or tumor cells.

**A method for diagnosing hepatocellular carcinoma in a subject**

The inventors developed a smoothing method for calculating and visualizing data from EWASs and demonstrated its utility for identifying highly accurate epigenomic signatures in DNA methylation analyses. By using this method, the inventors identified DNA methylation signature for diagnosing hepatocellular carcinoma.

Hepatocellular carcinoma (HCC) is the most common type of primary liver cancer. Hepatocellular carcinoma occurs most often in people with chronic liver diseases, such as cirrhosis caused by hepatitis B or hepatitis C infection. HCC may present with nonspecific symptoms such as abdominal pain, nausea, vomiting, or feeling tired. Some symptoms that are more associated with liver disease include yellow skin (also called jaundice), abdominal swelling due to fluid in the abdominal cavity, easy bruising from blood clotting abnormalities, loss of appetite, unintentional weight loss, abdominal pain, nausea, vomiting, or feeling tired.

The inventors identified in DNA sample of HCC patients, hypermethylated CpG sites within CpG islands located in the promoter regions of the genes selected from the group consisting of *RUNX3, KCNA3, DNM3, FOXL2/FOXL2NB, SHISA3, H3C7, BVES, EYA4, CELF2, PRKCB, SEPT9* and *LPAR2,* and in RNA non-coding region *MIR129-2.*

The hypermethylated CpG sites (CpG probes) and corresponding CpG islands identified in DNA samples of HCC patients are listed in Table 1 and are identified by an identification number of the chromosomic coordinates using the reference genome GRCh37 (Genome Reference Consortium Human Build 37 submitted on February 02, 2009) (hg37) (GenBank assembly accession: GCA_000001405.13).

| CpG probe | Chromosome | Position | *Gene Name* | CpG island | CpG island coordinates | Location of the GpG island |
|---|---|---|---|---|---|---|
| cg00117172 | 1 | 25255838 | *RUNX3* | CpG: 311 | Chr1: 25,255,528 - 25,259,005 (3.5 Kbp) | Promoter |
| cg06377278 | 1 | 25256369 | *RUNX3* | CpG: 311 | Chr1: 25,255,528 - 25,259,005 (3.5 Kbp) | Promoter |
| cg19270505 | 1 | 25256705 | *RUNX3* | CpG: 311 | Chr1: 25,255,528 - 25,259,005 (3.5 Kbp) | Promoter |
| cg11018723 | 1 | 25256939 | *RUNX3* | CpG: 311 | Chr1: 25,255,528 - 25,259,005 (3.5 Kbp) | Promoter |
| cg13629563 | 1 | 25256949 | *RUNX3* | CpG: 311 | Chr1: 25,255,528 - 25,259,005 (3.5 Kbp) | Promoter |
| cg22737001 | 1 | 25257029 | *RUNX3* | CpG: 311 | Chr1: 25,255,528 - 25,259,005 (3.5 Kbp) | Promoter |
| cg26421310 | 1 | 25257058 | *RUNX3* | CpG: 311 | Chr1: 25,255,528 - 25,259,005 (3.5 Kbp) | Promoter |

| | | | | | | |
|---|---|---|---|---|---|---|
| cg26013553 | 1 | 111217406 | *KCNA3* | CpG: 190 | Chr1: 111,216,245 - 111,217,937 (1.7 Kbp) | Promoter |
| cg11595545 | 1 | 111217497 | *KCNA3* | CpG: 190 | Chr1: 111,216,245 - 111,217,937 (1.7 Kbp) | Promoter |
| cg01423964 | 1 | 111217575 | *KCNA3* | CpG: 190 | Chr1: 111,216,245 - 111,217,937 (1.7 Kbp) | Promoter |
| cg06750832 | 1 | 111217691 | *KCNA3* | CpG: 190 | Chr1: 111,216,245 - 111,217,937 (1.7 Kbp) | Promoter |
| cg07808555 | 1 | 111217712 | *KCNA3* | CpG: 190 | Chr1: 111,216,245 - 111,217,937 (1.7 Kbp) | Promoter |
| cg02011074 | 1 | 171810468 | *DNM3* | CpG: 84 | Chr1: 171,810,468 - 171,811,325 (858 bp) | Promoter |
| cg26261793 | 1 | 171810543 | *DNM3* | CpG: 84 | Chr1: 171,810,468 - 171,811,325 (858 bp) | Promoter |
| cg06211893 | 1 | 171810778 | *DNM3* | CpG: 84 | Chr1: 171,810,468 - 171,811,325 (858 bp) | Promoter |
| cg05377226 | 1 | 171810910 | *DNM3* | CpG: 84 | Chr1: 171,810,468 - 171,811,325 (858 bp) | Promoter |
| cg23391785 | 1 | 171810972 | *DNM3* | CpG: 84 | Chr1: 171,810,468 - 171,811,325 (858 bp) | Promoter |
| cg26839871 | 1 | 171811299 | *DNM3* | CpG: 84 | Chr1: 171,810,468 - 171,811,325 (858 bp) | Promoter |
| cg10641721 | 3 | 138665006 | *FOXL2* | CpG: 230 | Chr3: 138,663,719 - 138,666,346 (2.6 Kbp) | Promoter (head-to-head genes) |
| cg05386493 | 3 | 138665654 | *FOXL2* | CpG: 230 | Chr3: 138,663,719 - 138,666,346 (2.6 Kbp) | Promoter (head-to-head genes) |
| cg18734304 | 3 | 138665816 | *FOXL2* | CpG: 230 | Chr3: 138,663,719 - 138,666,346 (2.6 Kbp) | Promoter (head-to-head genes) |
| cg13894046 | 3 | 138665930 | *FOXL2* | CpG: 230 | Chr3: 138,663,719 - 138,666,346 (2.6 Kbp) | Promoter (head-to-head genes) |
| cg25670330 | 3 | 138665984 | *FOXL2/ FOXL2NB (intergenic)* | CpG: 230 | Chr3: 138,663,719 - 138,666,346 (2.6 Kbp) | Promoter (head-to-head genes) |
| cg15720669 | 3 | 138666040 | *FOXL2/ FOXL2NB (intergenic)* | CpG: 230 | Chr3: 138,663,719 - 138,666,346 (2.6 Kbp) | Promoter (head-to-head genes) |
| cg04111064 | 3 | 138666048 | *FOXL2/ FOXL2NB (intergenic)* | CpG: 230 | Chr3: 138,663,719 - 138,666,346 (2.6 Kbp) | Promoter (head-to-head genes) |
| cg17802942 | 3 | 138666050 | *FOXL2/ FOXL2NB (intergenic)* | CpG: 230 | Chr3: 138,663,719 - 138,666,346 (2.6 Kbp) | Promoter (head-to-head genes) |
| cg17503456 | 3 | 138666054 | *FOXL2/ FOXL2NB (intergenic)* | CpG: 230 | Chr3: 138,663,719 - 138,666,346 (2.6 Kbp) | Promoter (head-to-head genes) |
| cg06186698 | 3 | 138666118 | *FOXL2NB* | CpG: 230 | Chr3: 138,663,719 - 138,666,346 (2.6 Kbp) | Promoter (head-to-head genes) |
| cg01379240 | 3 | 138666144 | *FOXL2NB* | CpG: 230 | Chr3: 138,663,719 - 138,666,346 (2.6 | Promoter (head-to- |

| | | | | | Kbp) | head genes) |
|---|---|---|---|---|---|---|
| cg13740698 | 4 | 42399384 | SHISA3 upstream | CpG: 153 | Chr4: 42,399,153 - 42,400,802 (1.6 Kbp) | Promoter |
| cg24084504 | 4 | 42399699 | SHISA3 | CpG: 153 | Chr4: 42,399,153 - 42,400,802 (1.6 Kbp) | Promoter |
| cg06269673 | 4 | 42399792 | SHISA3 | CpG: 153 | Chr4: 42,399,153 - 42,400,802 (1.6 Kbp) | Promoter |
| cg16862295 | 4 | 42399798 | SHISA3 | CpG: 153 | Chr4: 42,399,153 - 42,400,802 (1.6 Kbp) | Promoter |
| cg14866200 | 4 | 42399843 | SHISA3 | CpG: 153 | Chr4: 42,399,153 - 42,400,802 (1.6 Kbp) | Promoter |
| cg01565320 | 4 | 42399851 | SHISA3 | CpG: 153 | Chr4: 42,399,153 - 42,400,802 (1.6 Kbp) | Promoter |
| cg23684249 | 4 | 42399858 | SHISA3 | CpG: 153 | Chr4: 42,399,153 - 42,400,802 (1.6 Kbp) | Promoter |
| cg16094442 | 4 | 42400116 | SHISA3 | CpG: 153 | Chr4: 42,399,153 - 42,400,802 (1.6 Kbp) | Promoter |
| cg07638479 | 4 | 42400464 | SHISA3 | CpG: 153 | Chr4: 42,399,153 - 42,400,802 (1.6 Kbp) | Promoter |
| cg23841066 | 6 | 26250491 | H3C7 | CpG: 35 | Chr6: 26,250,437 - 26,250,827 (391 bp) | Promoter |
| cg15386964 | 6 | 26250685 | H3C7 | CpG: 35 | Chr6: 26,250,437 - 26,250,827 (391 bp) | Promoter |
| cg02578368 | 6 | 26250744 | H3C7 | CpG: 35 | Chr6: 26,250,437 - 26,250,827 (391 bp) | Promoter |
| cg14808890 | 6 | 26250826 | H3C7 | CpG: 35 | Chr6: 26,250,437 - 26,250,827 (391 bp) | Promoter |
| cg14159026 | 6 | 105584551 | BVES | CpG: 154 | Chr6: 105,584,149 - 105,585,621 (1.5 Kbp) | Promoter |
| cg10979880 | 6 | 105584689 | BVES | CpG: 154 | Chr6: 105,584,149 - 105,585,621 (1.5 Kbp) | Promoter |
| cg14314653 | 6 | 105584709 | BVES | CpG: 154 | Chr6: 105,584,149 - 105,585,621 (1.5 Kbp) | Promoter |
| cg13725782 | 6 | 105584718 | BVES | CpG: 154 | Chr6: 105,584,149 - 105,585,621 (1.5 Kbp) | Promoter |
| cg01513081 | 6 | 105584780 | BVES | CpG: 154 | Chr6: 105,584,149 - 105,585,621 (1.5 Kbp) | Promoter |
| cg26449787 | 6 | 133562087 | EYA4 | CpG: 138 | Chr6: 133,562,087 - 133,563,586 (1.5 Kbp) | Promoter |
| cg14017655 | 6 | 133562193 | EYA4 | CpG: 138 | Chr6: 133,562,087 - 133,563,586 (1.5 Kbp) | Promoter |
| cg06181518 | 6 | 133562196 | EYA4 | CpG: 138 | Chr6: 133,562,087 - 133,563,586 (1.5 Kbp) | Promoter |
| cg11518846 | 6 | 133562246 | EYA4 | CpG: 138 | Chr6: 133,562,087 - 133,563,586 (1.5 Kbp) | Promoter |
| cg20980055 | 6 | 133562258 | EYA4 | CpG: 138 | Chr6: 133,562,087 - | Promoter |

| | | | | | | |
|---|---|---|---|---|---|---|
| | | | | | 133,563,586 (1.5 Kbp) | |
| cg20286200 | 6 | 133562267 | EYA4 | CpG: 138 | Chr6: 133,562,087 - 133,563,586 (1.5 Kbp) | Promoter |
| cg05062333 | 6 | 133562269 | EYA4 | CpG: 138 | Chr6: 133,562,087 - 133,563,586 (1.5 Kbp) | Promoter |
| cg01162672 | 6 | 133562275 | EYA4 | CpG: 138 | Chr6: 133,562,087 - 133,563,586 (1.5 Kbp) | Promoter |
| cg01328892 | 6 | 133562332 | EYA4 | CpG: 138 | Chr6: 133,562,087 - 133,563,586 (1.5 Kbp) | Promoter |
| cg05085230 | 6 | 133562461 | EYA4 | CpG: 138 | Chr6: 133,562,087 - 133,563,586 (1.5 Kbp) | Promoter |
| cg08712932 | 6 | 133562463 | EYA4 | CpG: 138 | Chr6: 133,562,087 - 133,563,586 (1.5 Kbp) | Promoter |
| cg06132028 | 6 | 133562466 | EYA4 | CpG: 138 | Chr6: 133,562,087 - 133,563,586 (1.5 Kbp) | Promoter |
| cg11942956 | 6 | 133562470 | EYA4 | CpG: 138 | Chr6: 133,562,087 - 133,563,586 (1.5 Kbp) | Promoter |
| cg11664500 | 6 | 133562479 | EYA4 | CpG: 138 | Chr6: 133,562,087 - 133,563,586 (1.5 Kbp) | Promoter |
| cg14287112 | 6 | 133562485 | EYA4 | CpG: 138 | Chr6: 133,562,087 - 133,563,586 (1.5 Kbp) | Promoter |
| cg22871668 | 6 | 133562492 | EYA4 | CpG: 138 | Chr6: 133,562,087 - 133,563,586 (1.5 Kbp) | Promoter |
| cg14270292 | 6 | 133562494 | EYA4 | CpG: 138 | Chr6: 133,562,087 - 133,563,586 (1.5 Kbp) | Promoter |
| cg26656135 | 6 | 133562774 | EYA4 | CpG: 138 | Chr6: 133,562,087 - 133,563,586 (1.5 Kbp) | Promoter |
| cg24176563 | 6 | 133562776 | EYA4 | CpG: 138 | Chr6: 133,562,087 - 133,563,586 (1.5 Kbp) | Promoter |
| cg01401376 | 6 | 133563342 | EYA4 | CpG: 138 | Chr6: 133,562,087 - 133,563,586 (1.5 Kbp) | Promoter |
| cg21607030 | 6 | 133563532 | EYA4 | CpG: 138 | Chr6: 133,562,087 - 133,563,586 (1.5 Kbp) | Promoter |
| cg10297491 | 10 | 11059677 | CELF2 | CpG: 131 | Chr10: 11,059,443 - 11,060,524 (1.1 Kbp) | Promoter |
| cg27041794 | 10 | 11059708 | CELF2 | CpG: 131 | Chr10: 11,059,443 - 11,060,524 (1.1 Kbp) | Promoter |
| cg17290701 | 10 | 11059714 | CELF2 | CpG: 131 | Chr10: 11,059,443 - 11,060,524 (1.1 Kbp) | Promoter |
| cg26328510 | 10 | 11059718 | CELF2 | CpG: 131 | Chr10: 11,059,443 - 11,060,524 (1.1 Kbp) | Promoter |
| cg03813164 | 10 | 11059725 | CELF2 | CpG: 131 | Chr10: 11,059,443 - 11,060,524 (1.1 Kbp) | Promoter |

| | | | | | | |
|---|---|---|---|---|---|---|
| cg12356890 | 10 | 11059727 | *CELF2* | CpG: 131 | Chr10: 11,059,443 - 11,060,524 (1.1 Kbp) | Promoter |
| cg11472279 | 10 | 11059733 | *CELF2* | CpG: 131 | Chr10: 11,059,443 - 11,060,524 (1.1 Kbp) | Promoter |
| cg11002404 | 10 | 11060038 | *CELF2* | CpG: 131 | Chr10: 11,059,443 - 11,060,524 (1.1 Kbp) | Promoter |
| cg04088969 | 10 | 11060048 | *CELF2* | CpG: 131 | Chr10: 11,059,443 - 11,060,524 (1.1 Kbp) | Promoter |
| cg15556502 | 11 | 43602845 | *MIR129-2*(ncRNA) | CpG: 61 | Chr11: 43,602,546 - 43,603,215 (670 bp) | Encompass |
| cg14416371 | 11 | 43602847 | *MIR129-2*(ncRNA) | CpG: 61 | Chr11: 43,602,546 - 43,603,215 (670 bp) | Encompass |
| cg14944647 | 11 | 43602857 | *MIR129-2*(ncRNA) | CpG: 61 | Chr11: 43,602,546 - 43,603,215 (670 bp) | Encompass |
| cg01939477 | 11 | 43602879 | *MIR129-2*(ncRNA) | CpG: 61 | Chr11: 43,602,546 - 43,603,215 (670 bp) | Encompass |
| cg16407471 | 11 | 43602914 | *MIR129-2*(ncRNA) | CpG: 61 | Chr11: 43,602,546 - 43,603,215 (670 bp) | Encompass |
| cg05376374 | 11 | 43602920 | *MIR129-2*(ncRNA) | CpG: 61 | Chr11: 43,602,546 - 43,603,215 (670 bp) | Encompass |
| cg03365311 | 11 | 43602965 | *MIR129-2*(ncRNA) | CpG: 61 | Chr11: 43,602,546 - 43,603,215 (670 bp) | Encompass |
| cg04279973 | 16 | 23846968 | *PRKCB* | CpG: 108 | Chr16: 23,846,942 - 23,848,102 (1.2 Kbp) | Promoter |
| cg03306374 | 16 | 23847325 | *PRKCB* | CpG: 108 | Chr16: 23,846,942 - 23,848,102 (1.2 Kbp) | Promoter |
| cg03217795 | 16 | 23847556 | *PRKCB* | CpG: 108 | Chr16: 23,846,942 - 23,848,102 (1.2 Kbp) | Promoter |
| cg05436658 | 16 | 23847568 | *PRKCB* | CpG: 108 | Chr16: 23,846,942 - 23,848,102 (1.2 Kbp) | Promoter |
| cg03156893 | 16 | 23847675 | *PRKCB* | CpG: 108 | Chr16: 23,846,942 - 23,848,102 (1.2 Kbp) | Promoter |
| cg09507526 | 16 | 23847816 | *PRKCB* | CpG: 108 | Chr16: 23,846,942 - 23,848,102 (1.2 Kbp) | Promoter |
| cg21370856 | 16 | 23848003 | *PRKCB* | CpG: 108 | Chr16: 23,846,942 - 23,848,102 (1.2 Kbp) | Promoter |
| cg19554255 | 17 | 75369051 | *SEPT9* | CpG: 138 | Chr17: 75,368,689 - 75,370,506 (1.8 Kbp) | Promoter |
| cg16779463 | 17 | 75369055 | *SEPT9* | CpG: 138 | Chr17: 75,368,689 - 75,370,506 (1.8 Kbp) | Promoter |
| cg17300544 | 17 | 75369091 | *SEPT9* | CpG: 138 | Chr17: 75,368,689 - 75,370,506 (1.8 Kbp) | Promoter |
| cg03804136 | 17 | 75369219 | *SEPT9* | CpG: 138 | Chr17: 75,368,689 - 75,370,506 (1.8 Kbp) | Promoter |
| cg15044248 | 17 | 75369224 | *SEPT9* | CpG: 138 | Chr17: 75,368,689 - 75,370,506 (1.8 Kbp) | Promoter |
| cg02884239 | 17 | 75369228 | *SEPT9* | CpG: 138 | Chr17: 75,368,689 - 75,370,506 (1.8 Kbp) | Promoter |

| cg20275528 | 17 | 75369484 | SEPT9 | CpG: 138 | Chr17: 75,368,689 - 75,370,506 (1.8 Kbp) | Promoter |
|---|---|---|---|---|---|---|
| cg12783819 | 17 | 75369657 | SEPT9 | CpG: 138 | Chr17: 75,368,689 - 75,370,506 (1.8 Kbp) | Promoter |
| cg05184938 | 17 | 75369939 | SEPT9 | CpG: 138 | Chr17: 75,368,689 - 75,370,506 (1.8 Kbp) | Promoter |
| cg16692998 | 19 | 19739173 | LPAR2 | CpG: 128 | Chr19: 19,738,573 - 19,739,821 (1.2 Kbp) | Promoter |
| cg17419241 | 19 | 19739180 | LPAR2 | CpG: 128 | Chr19: 19,738,573 - 19,739,821 (1.2 Kbp) | Promoter |
| cg02362385 | 19 | 19739192 | LPAR2 | CpG: 128 | Chr19: 19,738,573 - 19,739,821 (1.2 Kbp) | Promoter |
| cg04395369 | 19 | 19739209 | LPAR2 | CpG: 128 | Chr19: 19,738,573 - 19,739,821 (1.2 Kbp) | Promoter |
| cg19306047 | 19 | 19739407 | LPAR2 | CpG: 128 | Chr19: 19,738,573 - 19,739,821 (1.2 Kbp) | Promoter |

**Table 1: hypermethylated CpG probes within CpG island in HCC patients.** CpG probes and CpG islands are identified by an identification number of the chromosomic coordinates using the reference genome GRCh37 (Genome Reference Consortium Human Build 37 submitted on February 02, 2009) (hg37) (GenBank assembly accession: GCA_000001405.13).

In a particular embodiment, the present disclosure relates to a method for diagnosing HCC in a subject comprising the step of determining the methylation level of at least one CpG site within at least one, preferably 2, 3, 4, 5, 6, 7, 8, 9, 10, 11, 12 or 13 of CpG islands selected from CpG islands located in the promoter region of the genes *RUNX3, KCNA3, DNM3, FOXL2/FOXL2NB, SHISA3, H3C7, BVES, EYA4, CELF2, PRKCB, SEPT9* and *LPAR2,* and in RNA non-coding region *MIR129-2* in DNA sample obtained from the subject.

In a particular embodiment, the present disclosure relates to a method for diagnosing HCC in a subject comprising the step of determining the methylation level of at least one CpG site within each of CpG islands located in the promoter region of the genes *RUNX3, KCNA3, DNM3, FOXL2/FOXL2NB, SHISA3, H3C7, BVES, EYA4, CELF2, PRKCB, SEPT9* and *LPAR2,* and in RNA non-coding region *MIR129-2* in DNA sample obtained from the subject.

In a preferred embodiment, said CpG islands are listed in the Table 1 and the present disclosure relates to a method for diagnosing HCC in a subject comprising the step of determining the methylation level of at least one CpG site within at least one, preferably 2, 3, 4, 5, 6, 7, 8, 9, 10, 11 or 12 CpG islands of a set of CpG islands, more preferably within each of CpG islands of a set of CpG islands comprising:

- CpG island localized from positions 25,255,528 to 25,259,005 of chromosome 1, GRCh37.p13 (genome reference consortium (February 2009), Ref Seq CGF_000001405.13) (CpG: 311) ;
- CpG island localized from positions 111,216,245 to 111,217,937 of chromosome 1, GRCh37.p13 (genome reference consortium (February 2009), Ref Seq CGF_000001405.13) (CpG: 190) ;
- CpG island localized from positions 171,810,468 to 171,811,325 of chromosome 1, GRCh37.p13 (genome reference consortium (February 2009), Ref Seq CGF_000001405.13) (CpG: 84) ;
- CpG island localized from positions 138,663,719 to 138,666,346 of chromosome 3, GRCh37.p13 (genome reference consortium (February 2009), Ref Seq CGF_000001405.13) (CpG: 230) ;
- CpG island localized from positions 42,399,153 to 42,400,802 of chromosome 4, GRCh37.p13 (genome reference consortium (February 2009), Ref Seq CGF_000001405.13) (CpG: 153) ;
- CpG island localized from positions 26,250,437 to 26,250,827 of chromosome 6, GRCh37.p13 (genome reference consortium (February 2009), Ref Seq CGF_000001405.13) (CpG: 35);
- CpG island localized from positions 105,584,149 to 105,585,621 of chromosome 6, GRCh37.p13 (genome reference consortium (February 2009), Ref Seq CGF_000001405.13) (CpG: 154) ;
- CpG island localized from positions 133,562,087 to 133,563,586 of chromosome 6, GRCh37.p13 (genome reference consortium (February 2009), Ref Seq CGF_000001405.13) (CpG: 138) ;
- CpG island localized from positions 11,059,443 to 11,060,524 of chromosome 10, GRCh37.p13 (genome reference consortium (February 2009), Ref Seq CGF_000001405.13) (CpG: 131) ;
- CpG island localized from positions 43,602,546 to 43,603,215 of chromosome 11, GRCh37.p13 (genome reference consortium (February 2009), Ref Seq CGF_000001405.13) (CpG: 61) ;
- CpG island localized from positions 23,846,942 to 23,848,102 of chromosome 16, GRCh37.p13 (genome reference consortium (February 2009), Ref Seq CGF_000001405.13) (CpG: 108) ;
- CpG island localized from positions 75,368,689 to 75,370,506 of chromosome 17, GRCh37.p13 (genome reference consortium (February 2009), Ref Seq CGF_000001405.13) (CpG: 138) and ;
- CpG island localized from positions 19,738,573 to 19,739,821 of chromosome 19, GRCh37.p13 (genome reference consortium (February 2009), Ref Seq CGF_000001405.13) (CpG: 128).

In a more preferred embodiment, the present disclosure relates to a method for diagnosing HCC in a subject comprising the step of determining the methylation level of at least one CpG site within at least one, preferably 2, 3, 4, 5, 6, 7, 8, 9, 10, 11 or 12 CpG islands of a set of CpG islands, more preferably within each of CpG islands as described above wherein said CpG sites are selected from the group consisting of CpG sites listed in Table 1.

In a preferred embodiment, the method for diagnosing an HCC in a subject comprises the step of determining the methylation level of:

- at least one CpG site(s) within CpG island localized from positions 25,255,528 to 25,259,005 of chromosome 1, GRCh37.p13 (genome reference consortium (February 2009), Ref Seq CGF_000001405.13), preferably at least one, more preferably 2, 3, 4, 5, 6 or 7 CpG site(s) selected from the group consisting of cg00117172, cg06377278, cg19270505, cg11018723, cg13629563, cg22737001 and cg26421310,
- at least one CpG sites(s) within CpG island localized from positions 111,216,245 to 111,217,937 of chromosome 1, GRCh37.p13 (genome reference consortium (February 2009), Ref Seq CGF_000001405.13) (CpG: 190), preferably at least one, more preferably at least 2, 3, 4 or 5 CpG site(s) selected from the group consisting of cg26013553, cg11595545, cg01423964, cg06750832 and cg07808555,
- at least one CpG sites(s) within CpG island localized from positions 171,810,468 to 171,811,325 of chromosome 1, GRCh37.p13 (genome reference consortium (February 2009), Ref Seq CGF_000001405.13) (CpG: 84), preferably at least one, more preferably at least 2, 3, 4, 5 or 6 CpG site(s) selected from the group consisting of cg02011074, cg26261793, cg06211893, cg05377226, cg23391785 and cg26839871,
- at least one CpG sites(s) within CpG island localized from positions 138,663,719 to 138,666,346 of chromosome 3, GRCh37.p13 (genome reference consortium (February 2009), Ref Seq CGF_000001405.13) (CpG: 230), preferably at least one, more preferably at least 2, 3, 4, 5, 6, 7, 8, 9, 10 or 11 CpG site(s) selected from the group consisting of: cg10641721, cg05386493, cg18734304, cg13894046, cg25670330, cg15720669, cg04111064, cg17802942, cg17503456, cg06186698 and cg01379240,
- at least one CpG sites(s) within CpG island localized from positions 42,399,153 to 42,400,802 of chromosome 4, GRCh37.p13 (genome reference consortium (February 2009), Ref Seq CGF_000001405.13) (CpG: 153), preferably at least one, more preferably at least 2, 3, 4, 5, 6, 7, 8 or 9 CpG site(s) selected from the group consisting of: cg13740698, cg24084504, cg06269673, cg16862295, cg14866200, cg01565320, cg23684249, cg16094442 and cg07638479,
- at least one CpG sites(s) within CpG island localized from positions 26,250,437 to 26,250,827 of chromosome 6, GRCh37.p13 (genome reference consortium (February 2009), Ref Seq CGF_000001405.13) (CpG: 35), preferably at least one, more preferably at least 2, 3 or 4 CpG site(s) selected from the group consisting of: cg23841066, cg15386964, cg02578368 and cg14808890,
- at least one CpG sites(s) within CpG island localized from positions 105,584,149 to 105,585,621 of chromosome 6, GRCh37.p13 (genome reference consortium (February 2009), Ref Seq CGF_000001405.13) (CpG: 154), preferably at least one, more preferably at least 2, 3, 4 or 5 CpG site(s) selected from the group consisting of: cg14159026, cg10979880, cg14314653, cg13725782 and cg01513081,
- at least one CpG sites(s) within CpG island localized from positions 133,562,087 to 133,563,586 of chromosome 6, GRCh37.p13 (genome reference consortium (February 2009), Ref Seq CGF_000001405.13) (CpG: 138), preferably at least one, more preferably at least 2, 3, 4, 5, 6, 7, 8, 9, 10, 12, 15, 17, 20 or 21 CpG site(s) selected from the group consisting of: cg26449787, cg14017655, cg06181518, cg11518846, cg20980055, cg20286200, cg05062333, cg01162672, cg01328892, cg05085230, cg08712932, cg06132028, cg11942956, cg11664500, cg14287112, cg22871668, cg14270292, cg26656135, cg24176563, cg01401376, cg21607030,
- at least one CpG sites(s) within CpG island localized from positions 11,059,443 to 11,060,524 of chromosome 10, GRCh37.p13 (genome reference consortium (February 2009), Ref Seq CGF_000001405.13) (CpG: 131), preferably at least one, more preferably at least 2, 3, 4, 5, 6, 7, 8 or 9 CpG site(s) selected from the group consisting of:

cg10297491, cg27041794, cg17290701, cg26328510, cg03813164, cg12356890, cg11472279, cg11002404 and cg04088969,

- at least one CpG sites(s) within CpG island localized from positions 43,602,546 to 43,603,215 of chromosome 11, GRCh37.p13 (genome reference consortium (February 2009), Ref Seq CGF_000001405.13) (CpG: 61), preferably at least one, more preferably at least 2, 3, 4, 5, 6, or 7 CpG site(s) selected from the group consisting of: cg15556502, cg14416371, cg14944647, cg01939477, cg16407471, cg05376374 and cg03365311,

- at least one CpG sites(s) within CpG island localized from positions 23,846,942 to 23,848,102 of chromosome 16, GRCh37.p13 (genome reference consortium (February 2009), Ref Seq CGF_000001405.13) (CpG: 108), preferably at least one, more preferably at least 2, 3, 4, 5, 6, or 7 CpG site(s) selected from the group consisting of: cg04279973, cg03306374, cg03217795, cg05436658, cg03156893, cg09507526, cg21370856,

- at least one CpG sites(s) within CpG island localized from positions 75,368,689 to 75,370,506 of chromosome 17, GRCh37.p13 (genome reference consortium (February 2009), Ref Seq CGF_000001405.13) (CpG: 138), preferably at least one, more preferably at least 2, 3, 4, 5, 6, 7, 8 or 9 CpG site(s) selected from the group consisting of: cg19554255, cg16779463, cg17300544, cg03804136, cg15044248, cg02884239, cg20275528, cg12783819 and cg05184938, and/or ;

- at least one CpG sites(s) within CpG island localized from positions 19,738,573 to 19,739,821 of chromosome 19, GRCh37.p13 (genome reference consortium (February 2009), Ref Seq CGF_000001405.13) (CpG: 128), preferably at least one, more preferably at least 2, 3, 4, 5, 6, 7, 8 or 9 CpG site(s) selected from the group consisting of: cg16692998, cg17419241, cg02362385, cg04395369 and cg19306047.

The determination of the methylation level may be performed using any method known in the art to be suitable for assessing the methylation of cytosine residues in DNA. Such methods are known in the art and have been described; and one skilled in the art will know how to select the most suitable method depending on the number of samples to be tested, the quantity of sample available, and the like.

For example, methylation can be measured by employing a restriction enzyme-based technology, which utilizes methylation-sensitive restriction endonucleases for the differentiation between methylated and unmethylated cytosines. Restriction enzyme-based technologies include, for example, restriction digest with methylation-sensitive restriction enzymes followed by nucleic acid sequencing (e.g., massively parallel or Next Generation sequencing), Southern blot analysis, real-time PCR, restriction landmark genomic scanning (RLGS) or differential methylation hybridization (DMH).

In some embodiments that employ methylation-sensitive restriction enzymes, a post-digest PCR amplification step is added wherein a set of two oligonucleotide primers, one on each side of the methylation-sensitive restriction site, is used to amplify the digested genomic DNA. PCR products are produced and detected for templates that were not restricted (e.g., due to the presence of a methylated restriction site) whereas PCR products are not produced where digestion of the subtended methylation-sensitive restriction enzyme site occurs. Techniques for restriction enzyme-based analysis of genomic methylation are well known in the art and include the following: differential methylation hybridization (DMH) (Huang et al., 1999, Human Mol. Genet. 8, 459-70); Not I-based differential methylation hybridization (for example, WO02/086163A1); restriction landmark genomic scanning (RLGS) (Plass et al., 1999, Genomics 58:254-62); methylation-sensitive arbitrarily primed PCR (AP-PCR) (Gonzalgo et al., 1997, Cancer Res. 57: 594-599); methylated CpG site amplification (MCA) (Toyota et al., 1999, Cancer Res. 59: 2307-2312).

In a preferred embodiment, the methods rely on the prior treatment of DNA with sodium bisulfite. This treatment leads to the conversion of unmethylated cytosine to uracil, while methylated cytosine remains unchanged (Clark et al., Nucleic Acids Res., 1994, 22: 2990-2997). This change in the DNA sequence following bisulfite conversion can be detected using a variety of methods, including PCR amplification followed by DNA hybridization on a microarray of probes. The protocol described by Frommer et al. (PNAS USA, 1992, 89: 1827-183 1) has been widely used for sodium bisulfite treatment of DNA, and a variety of commercial kits are now available for this purpose.

Techniques for the analysis of bisulfite-treated DNA can employ methylation-sensitive primers for the analysis of CpG methylation status with isolated genomic DNA, for example, as described by Herman et al., 1996, Proc. Natl. Acad. Sci. USA 93:9821-9826, or U.S. Pat. Nos. 5,786,146 or 6,265,171). Methylation sensitive PCR (MSP) allows for the detection of a specific methylated CpG position within, for example, the regulatory region of a gene. The DNA of interest is treated such that methylated and unmethylated cytosines are differentially modified, for example, by bisulfite treatment, in a manner discernable by their hybridization behavior. PCR primers specific to each of the methylated and unmethylated states of the DNA are used in PCR amplification. Products of the amplification reaction are then detected, allowing for the deduction of the methylation status of the CpG position within the genomic DNA. Other methods for the analysis of bisulfite-treated DNA include methylation-sensitive single nucleotide primer extension (Ms-SNuPE) (see, for example, Gonzalgo & Jones, 1997; Nucleic Acids Res. 25:2529-253 1, or U.S. Patent 6,251,594), or the use of real-time PCR based methods, such as the art-recognized fluorescence-based real-time PCR technique MethyLight™ (see, for example, Eads et al., 1999; Cancer Res. 59:2302-2306, U.S. Pat. No. 6,331,393 or Heid et al., 1996, Genome Res. 6:986-994). It will be understood that a variety of methylation assay methods can be used for the determination of the methylation status of particular genomic CpG positions. Methods that employ bisulfite conversion include, for example, bisulfite sequencing, methylation-specific PCR, methylation-sensitive single nucleotide primer extension (Ms-SnuPE), MALDI mass spectrometry and methylation-specific oligonucleotide arrays, for example, as described in U.S. Pat. No. 7,61 1,869 or International Patent Application WO2004/05 1224).

In a preferred embodiment, the methylation level of a plurality of CpG sites in a sample is detected using an array or chip of probes.

In such embodiments, a plurality of different probe molecules can be attached to a substrate or otherwise spatially distinguished in an array. Said probes are labeled, for example, with a fluorescent molecule, and the level of methylation can be determined by calculating the ratio of the fluorescence signals from the methylated versus unmethylated sites.

A variety of commercially available array-based products to detect methylation can be used, including, for example, the MethylationEPIC™ BeadChip™ (Illumina, Inc., San Diego, CA), which allows interrogation of over 850,000 methylation sites quantitatively across the human genome at single-nucleotide resolution.

Typically, the methylation level of said CpG sites as described above is compared to a control value.

As used herein, the term "control value" refers to the methylation level (e.g., beta-value) in a biological sample obtained from a general population or from a selected population of subjects. For example, the general population may comprise apparently healthy subjects, such as individuals who have not previously had any sign or symptoms indicating the presence of hepatocellular carcinoma. The term "healthy subjects" as used herein refers to a population of subjects who do not suffer from any known condition, and in particular, who are not affected with any cancer. The control value can be a threshold value, or a range. The control value can be established based upon comparative measurements between apparently healthy subjects and subjects with established cancer.

In some embodiments, the methylation level (e.g., beta value) of each CpG site is higher than a control value, preferably higher than 1.2, 1.5, 2 or 5, preferably 10, even more preferably 15, even more preferably 20, 30, 40, 50, 60, 70, 80, 90, 95 or 99. The higher methylation level of CpG sites within the set of CpG islands as described above is indicative that said subject had hepatocellular carcinoma.

Examples of biological samples suitable for use in the practice of the present disclosure include, but are not limited to, bodily fluids, e.g., blood samples (e.g., blood smears), and cerebrospinal fluid, brain tissue samples or bone marrow tissue samples such as tissue or fine needle biopsy samples. Biological samples may also include sections of tissues such as frozen sections taken for histological purposes. The term "biological sample" also encompasses any material derived by processing a biological sample. Derived materials include, but are not limited to, cells (or their progeny) isolated from the sample. In a preferred embodiment, said sample is nucleic acid molecules (e.g., DNA) extracted from the sample. Biological sample may also be circulating free DNA (cfDNA) which are degraded DNA fragments released to the blood plasma. cfDNA can be derived from healthy or tumor cells.

**Therapeutic use**

In connection with the above method for diagnosing hepatocellular carcinoma, the present disclosure relates to a cancer drug for use in the treatment of hepatocellular carcinoma. In some preferred embodiments, said cancer drug is used in a patient previously diagnosed as having hepatocellular carcinoma using the method for diagnosing hepatocellular carcinoma as described above.

The present invention relates also to a method of treating hepatocellular carcinoma in a patient in need thereof comprising administering to said patient a therapeutically effective amount of a cancer drug, preferably said cancer agent is administered to the patient previously diagnosed as having hepatocellular carcinoma using the method for diagnosing hepatocellular carcinoma as described above.

As used herein, a "therapeutically effective amount" or an "effective amount" means the amount of a composition that, when administered to a subject for treating a state, disorder or condition is sufficient to effect a treatment. The therapeutically effective amount will vary depending on the compound, formulation or composition, the disease and its severity and the age, weight, physical condition and responsiveness of the subject to be treated.

"Treating hepatocellular carcinoma (HCC) includes, without limitation, reducing the number of cancer cells or the size of a tumor in the patient, reducing the progression of cancer to a more aggressive form (i.e., maintaining the cancer in a form that is susceptible to a therapeutic agent), reducing the proliferation of cancer cells or reducing the speed of tumor growth, killing of cancer cells, reducing metastasis of cancer cells or reducing the likelihood of recurrence of cancer in a subject. Treating a subject as used herein refers to any type of treatment that imparts a benefit to a subject afflicted with cancer or at risk of developing cancer or facing a cancer recurrence. Treatment includes improvement in the condition of the subject (e.g., in one or more symptoms), delay in the progression of the disease, delay in the onset of symptoms or slowing the progression of symptoms, etc.

As used herein, "drug" or "therapeutic agent" refers to a compound or agent that provides a desired biological or pharmacological effect when administered to a human or animal, particularly results in an intended therapeutic effect or response on the body to treat or prevent conditions or diseases. Therapeutic agents include any suitable biologically active chemical compounds, biologically derived components such as, for example, small molecules, cells, proteins, peptides, antibodies, enzymes, polynucleotides, and radiochemical therapeutic agents, such as radioisotopes.

The cancer drug is any drug for chemotherapy or targeted therapy. Targeted therapy includes the use of "targeted" drugs such as small molecule inhibitors or neutralizing monoclonal antibodies, that target proteins that are abnormally expressed in cancer cells and that are essential for their growth such as for example receptor and non-receptor tyrosine kinases, growth factors, hormone receptors, and others. Chemotherapy includes the use of cytotoxic anti-neoplastic agents, such as alkylating agents, anti-metabolites, anti-microtubule agents, Topoisomerase inhibitors, cytotoxic antibiotics and others. Examples of chemotherapeutic drugs include with no limitations: Capecitabine, 5-FU, docetaxel, SN-38, CPT11, cisplatin, carboplatin, etc.

The cancer drugs described herein may be administered by any means known to those skilled in the art, including, without limitation, intravenously, orally, intra-tumoral, intra-lesional, intradermal, topical, intraperitoneal, intramuscular, parenteral, subcutaneous and topical administration. Thus the compositions may be formulated as an injectable, topical, ingestible, or suppository formulation. Administration of the compounds or therapeutic agents to a subject in accordance with the present invention may exhibit beneficial effects in a dose-dependent manner. Thus, within broad limits, administration of larger quantities of the compositions is expected to achieve increased beneficial biological effects than administration of a smaller amount. Moreover, efficacy is also contemplated at dosages below the level at which toxicity is seen.

It will be appreciated that the specific dosage of cancer drugs administered in any given case will be adjusted in accordance with the composition being administered, the volume of the composition that can be effectively delivered to the site of administration, the condition of the subject, and other relevant medical factors that may modify the activity of the compositions or the response of the subject, as is well known by those skilled in the art.

For example, the specific dose of cancer drugs for a particular subject depends on age, body weight, general state of health, diet, the timing and mode of administration, the rate of excretion, medicaments used in combination and the severity of the particular disorder to which the therapy is applied. Dosages for a given patient can be determined using conventional considerations, e.g., by customary comparison of the differential activities of the compositions described herein and of a known agent, such as by means of an appropriate conventional pharmacological protocol. The compositions can be given in a single dose schedule, or in a multiple-dose schedule.

Suitable dosage ranges for a cancer drug may be of the order of several hundred micrograms of the agent with a range from about 0.001 to 10 mg/kg/day, preferably in the range from about 0.01 to 1 mg/kg/day.

**Kit**

The present disclosure also relates to a kit comprising or consisting of a set of probes for measuring the methylation level of at least one CpG site within each of CpG islands listed in Table 1, in particular a set of probes which each hybridizes with a nucleic acid sequence comprising at least one CpG site within each of CpG islands listed in Table 1, in particular wherein said CpG sites are selected from the group of CpG sites listed in Table 1.

In a particular embodiment, said kit comprises an array of said probes, in particular wherein a plurality of probes is attached to a substrate. The length and complexity of the probe fixed onto the target element is not critical to the invention. One skilled in the art can adjust these factors to provide optimum hybridization and signal production for a given hybridization procedure, and to provide the required resolution among different genes or genomic locations.

Probes according to the present disclosure may be designed according to the specific CpG sites as described above, in particular a probe that hybridizes to a cytosine that is methylated and not modified by bisulfite pre-treatment of DNA and a probe that hybridizes to an unmethylated cytosine that is modified in uracil by bisulfite pre-treatment of DNA.

The kit is particularly suitable for microarray technology that utilizes nucleic acid hybridization techniques and advancements in computing technology. Microarray is a compact device that contains the well-defined immobilized capture nucleic acid sequences (probes) assembled in an addressable format. The probes are attached to a glass or plastic surface. The microarray using the probes of the kit according to the present disclosure, is used to identify simultaneously the CpG sites as described above with cytosine methylated, and/or uracile unmethylated by hybridization between the DNA sample of the subject and the probes on the microarray.

The kits can additionally include bisulfite reagents and instructional materials describing when and how to use the kit contents. The kits can also include one or more of the following: various labels or labeling agents to facilitate the detection of the probes, reagents for the hybridization including buffers, sampling devices including fine needles, swabs, aspirators and the like, positive and negative controls and so forth.

The invention will now be exemplified with the following examples, which are not limitative, with reference to the attached drawings in which:

**EXAMPLES**

**METHODS**

**Description of EWAS datasets and overview of the study design**

The inventors conducted a three-stage EWAS of HCC in 353 cases and 233 controls originating from three independent cohorts. In stage 1 (initial study), the inventors analyzed the epigenome-wide DNA methylome profiles generated from 27 HCC, 100 non-HCC, and control liver tissue samples using the Infinium Human Methylation 450K BeadChip. In stage 2 (replication study #1), the inventors performed in silico epigenome association study using epigenome-wide DNA methylome profiles generated from 26 HCC and 83 non-HCC liver tissue samples. In stage 3 (replication study #2), the inventors used the same approach as in replication #1 on 306 HCC and 50 control liver tissue samples.

In the initial study, the inventors performed an EWAS to identify the top significant epigenome signatures associated with the HCC phenotype. In the replication studies, the inventors performed epigenome association analyses on the 105 top significant CpG probes identified in the initial study. In the third step, the inventors performed a meta-analysis to combine to estimate the epigenome association considering the three EWASs. In parallel, the inventors derived an epigenomic score based on the top significant CpG signatures, the HCC Epigenome Score. The inventors assessed the diagnostic accuracy HCC Epigenome Score in association with the HCC phenotype using ROC analysis and dose-response probit regression analysis.

**Statistical analysis**

*Quality controls of methylome array data*

The inventors visually inspected the genome-wide distribution of the CpG probes according to their β value. The inventors assessed population stratification according to the whole methylome landscape by performing numeric principal component analysis on normalized beta values of each CpG probe across the methylation array. The top ten principal components (eigenvectors) were calculated with their respective eigenvalue.

**DNA methylome analyses and elaboration of the 'HCC Epigenome Score'**

Methylation arrays allow the assessment of DNA methylation status at a single-nucleotide resolution (CpG probe) through a β value ranging from 0 (fully unmethylated CpG) to 1 (fully methylated CpG). According to the ENCODE project, any β value ≥0.6 corresponds to a fully methylated CpG probe, any β value ≤0.2 corresponds to an unmethylated CpG probe, and any β value >2 and <0.6 corresponds to a hemimethylated CpG probe (Consortium EP. Nature 2012;489:57-74). Based on these validated thresholds, the inventors propose a pseudo-genotypic transformation of β values accordingly: unmethylated (AA) for β values ≤0.2; hemimethylated, (AB) for β values >2 and <0.6; fully-methylated (BB) for β values ≥0.6. This approach helps to minimize the variance of the continuous variables (β values) within strata as suggested by the Dalenius-Hodges rule (Dalenius T, Hodges Jr JL. J Am Stat Assoc 1959;54:88-101). Consequently, the pseudo-genotypic approach for methylation analyses enables controlling for spurious associations and genomic inflation without loss of data and allows using statistical methods developed for genotypic data.

In the initial study, the inventors assessed the association between the methylation profile of each CpG probe and the HCC status using Fischer's exact test. The inventors used the smoothing method to look for the top significant epigenome signatures, as described below. The inventors elaborated the "HCC Epigenome Score" by calculating the mean beta-values obtained for the top significant CpG probes for each sample.

**Smoothing method for calculating and visualizing data from EWAS**

The smoothing method relies on the DNA co-methylation pattern, which is the correlation between adjacent CpG sites. The first description of the co-methylation pattern of CpG sites was reported by Eckhardt *et al.* using bisulfite DNA sequencing to generate high-resolution methylation profiles of human chromosomes 6, 20, and 22, providing a resource of about 1.9 million CpG methylation values derived from 12 different tissues (Eckhardt, F. et al. Nat Genet 38, 1378-1385, (2006)). In this landmark study, over 90% of CpG sites within 50 bases had the same methylation status. Taking into account this assumption, the inventors proposed the conversion of the nominal - Log10(*P*-value) obtained for each CpG probe at the genome-wide level to smoothed - Log10(*P*-values) using a pre-specified window width (*w*) value ranging between 1 and 3 (Smoothed[$P_{k,w}$]) **(Figure 1).** The inventors used a symmetric smoothing method so that the Smoothed($P_{k,w}$) of each CpG probe corresponds to the mean of the -Log10(*P*-values) of the central CpG probe (k) and those located on either side of the central CpG probe within the limit of the pre-defined window width, through a sliding window approach, according to the following formula:

$$Smoothed(Pk, w) = \sum_{n=k-w}^{n=k+w} (-Log10[P - value]) \div (2w + 1)$$

, where $k$ corresponds to the central CpG probe and w to the number of CpG probes on either side of the central CpG probe (window width). The Infinium Human Methylation 450k BeadChip array encompasses 482,421 CpG probes and 26,658 CpG islands with an average number of 5.63 CpG probes per CpG island (Bibikova, M. et al. Genomics 98, 288-295, (2011)). Considering this resolution, the inventors estimated the optimal smoothing width at 2 since this value corresponds to a total number of 5 CpG sites ($2w + 1$) to calculate the Smoothed($P_{k,w}$) value.

To assess the magnitude of signal enhancement at a given top significant locus, the inventors used several metrics, including the signal-to-noise ratio (SNR), variance reduction, and variance ratio (VR). For a given top significant locus, the signal-to-noise ratio (SNR) was defined as the ratio of the power of a signal (meaningful information at the top locus) and the power of background noise (unwanted signal in the remaining genomic regions at the genome-wide level) according to the following formula (Bushberg, J. T. & Boone, J. M. (Lippincott Williams & Wilkins, 2011)) :

$$\text{Signal} - \text{to} - \text{noise ratio (SNR)} = \frac{\mu \text{ (signal)}}{\sigma \text{ (noise)}}$$

, where '$\mu$ (signal)' corresponds to the mean of Smoothed($P_{k,w}$) of the CpG probes located in the analyzed top significant locus and '$\sigma$ (noise)' to the standard deviation of Smoothed($P_{k,w}$) of all the CpG probes located outside the analyzed top significant locus at the genome-wide level, using the same window width value ($w$).

The inventors calculated the variance ratio according to the following formula:

$$\text{Variance ratio (VR)} = \frac{VAR \text{ (Smoothed}[Pk, w]) \text{ (signal)}}{VAR \text{ (Smoothed}[Pk, w]) \text{ (noise)}}$$

, where 'VAR (Smoothed[$P_{k,w}$]) (signal)' corresponds to the variance calculated from the Smoothed[$P_{k,w}$] of the CpG probes located in the studied genomic region and 'VAR (Smoothed[$P_{k,w}$]) (noise)' to the variance calculated from the Smoothed[$P_{k,w}$] of all the CpG probes located outside the studied genomic region at the genome-wide level, using the same window width value (w).

The inventors reported the results of epigenome-wide association studies using epi-Manhattan plots for nominal and smoothing transformed -Log10(P-values). In the epi-Manhattan illustrating the smoothing transformation of -Log10(P-values), the inventors used ($2 \times \sigma^2$) as a significance threshold value, where $\sigma^2$ corresponds to 'VAR (Smoothed[$Pk,w$]) (noise)', as previously described. The smoothing method and all statistical analyses were performed using the SNP & Variation Suite (v8.8.1; Golden Helix, Inc., Bozeman, MT, USA) and MedCalc, version 19.5.3 (MedCalc Software, Ostend, Belgium).

**Hierarchical clustering analysis**

To better visualize the distribution of CpG absolute beta values between HCC and non-HCC samples, the inventors used dendrogram with heat map representation according to the following criteria: distance metric for top dendrogram: Euclidean; linkage algorithm for top dendrogram: weighted; distance metric for side dendrogram: Euclidean; and linkage algorithm for side dendrogram: weighted.

**Receiver operating characteristic analysis**

The inventors assessed the diagnostic accuracy of the HCC Epigenome Score by performing receiver operating characteristic (ROC) analysis, according to DeLong *et al.* (DeLong ER, et al. Biometrics 1988;44:837-845). The continuous variable was the HCC Epigenome Score and the classification variable was the HCC phenotype. The inventors reported the area under the receiver operating characteristic curve (AUROC) and the associated P-value. The inventors applied the exact binomial method to estimate the 95% confidence intervals (CIs) of the AUROC. The optimal diagnostic cut-off was defined using the Youden index J. Bias-corrected and accelerated (BC$_a$)-bootstrap interval after 10,000 iterations for the Youden index and its associated values was performed (Efron B, Tibshirani RJ. An Introduction to the Bootstrap: Taylor & Francis, 1994). Other diagnostic accuracy measures included: sensitivity, specificity, positive and negative likelihood ratios, positive and negative predictive values.

**Meta-analysis**

The inventors used the generic inverse variance method to estimate the pooled effect size from the three studies (initial, replication #1, and replication #2) for the epigenome association analyses on the 105 top significant CpG probes. Using the same meta-analysis approach, the inventors also assessed the pooled AUROC from the three datasets. The inventors calculated the pooled effect size based on estimates and standard errors. In the inverse variance method, the weight given to each study is the inverse of the variance of the effect estimate. This choice of weight minimizes the imprecision (uncertainty) of the pooled effect estimate. The overall summary from the meta-analysis was calculated by combining all studies, and the meta-analysis was performed using four different random-effect model estimators: DerSimonian and Laird, maximum likelihood, empirical Bayes, and restricted maximum likelihood. The calculated summary effect was denoted by the solid diamond at the bottom of the forest plots, the width of which represents the 95% CI. The inventors assessed the statistical significance for heterogeneity using the χ2-based Q and $I^2$ statistics. Heterogeneity was considered significant if the *P*-value was < 0.1 and the $I^2$ > 50% (Higgins JP, et al. BMJ 2003;327:557-560).

**RESULTS**

**Initial study**

On the 127 DNA methylome profiles assessed, 121 were of optimal quality and were used for statistical analyses. The PCA analysis on genome-wide DNA methylome profiles revealed two clusters corresponding to HCC and non-HCC samples **(Figure 2).** The epigenome-wide association study highlighted 13 top loci associated with the HCC phenotype (*RUNX3, KCNA3, DNM3, FOXL2/FOXL2NB, SHISA3, H3C7, BVES, EYA4, CELF2, MIR129-2, PRKCB, SEPT9,* and *LPAR2*) **(Figure 3).** All the thirteen loci corresponded to CpG islands and covered 105 CpG probes detailed in **Table 1.** The CpG islands in these 13 loci exhibited a hemimethylated profile in HCC samples and a completely unmethylated profile in non-HCC samples. The 105 CpG probes were highly discriminant between HCC and non-HCC samples in the hierarchical clustering analysis. The inventors elaborated the "HCC Epigenome Score" by calculating the mean beta-values obtained for the 105 CpG probes for each sample. In ROC analysis, the HCC Epigenome Score had an AUROC of 0.960 (95% CI, 0.788-100; *P* < 0.0001) to detect HCC samples **(Figure 4).**

**Replication studies #1 and #2 and meta-analysis**

The inventors performed in silico replication studies on two independent DNA methylome datasets using the 105 CpG probes of the HCC Epigenome Score. All the 13 loci were significantly associated with the HCC phenotype in both replication studies **(Figure 5).** Consistently, the random effect meta-analysis confirmed the significance of the 13 top loci **(Table 2** and **Figure 5).**

**Table 2. Random-effect meta-analysis for the association between the methylation profile of the 105 CpG probes of the HCC Epigenome Score and the HCC phenotype**

| Marker | Chr | Position | Overall *P*-Value | Overall, OR (9% CI) | RE meta-analysis, *P*-Value | RE meta-analysis, OR (95% CI) | Cochran's *Q, P*-Value | $I^2$ |
|---|---|---|---|---|---|---|---|---|
| cg00117172 | 1 | 25255838 | 2.81E-28 | 1.85 (1.66-2.07) | 1.38E-06 | 1.97 (1.5-2.6) | 3.25E-03 | 83% |
| cg06377278 | 1 | 25256369 | 9.14E-47 | 2.23 (2-2.49) | 3.23E-16 | 2.28 (1.87-2.78) | 5.24E-02 | 66% |
| cg19270505 | 1 | 25256705 | 1.44E-36 | 2.02 (1.81-2.26) | 2.55E-12 | 2.09 (1.7-2.58) | 3.98E-02 | 69% |
| cg11018723 | 1 | 25256939 | 4.93E-15 | 1.55 (1.39-1.73) | 4.98E-04 | 1.65 (1.25-2.19) | 2.47E-03 | 83% |
| cg13629563 | 1 | 25256949 | 7.01E-29 | 1.86 (1.67-2.08) | 5.88E-10 | 1.89 (1.54-2.31) | 4.78E-02 | 67% |
| cg22737001 | 1 | 25257029 | 7.55E-34 | 1.97 (1.76-2.2) | 1.18E-07 | 2.09 (1.59-2.74) | 3.77E-03 | 82% |

(continued)

| Marker | Chr | Position | Overall P-Value | Overall, OR (9% CI) | RE meta-analysis, P-Value | RE meta-analysis, OR (95% CI) | Cochran's Q, P-Value | I² |
|---|---|---|---|---|---|---|---|---|
| cg26421310 | 1 | 25257058 | 5.05E-22 | 1.71 (1.54-1.91) | 4.65E-05 | 1.84 (1.37-2.46) | 1.63E-03 | 84% |
| cg26013553 | 1 | 11121740 6 | 1.82E-38 | 2.06 (1.85-2.3) | 1.69E-10 | 2.15 (1.7-2.72) | 1.60E-02 | 76% |
| cg11595545 | 1 | 11121749 7 | 1.66E-72 | 2.73 (2.45-3.05) | 3.57E-22 | 2.8 (2.27-3.45) | 3.83E-02 | 69% |
| cg01423964 | 1 | 11121757 5 | 1.32E-30 | 1.9 (1.7-2.12) | 1.82E-14 | 1.94 (1.64-2.3) | 1.14E-01 | 54% |
| cg06750832 | 1 | 11121769 1 | 2.17E-42 | 2.14 (1.92-2.39) | 1.61E-38 | 2.14 (1.91-2.41) | 3.39E-01 | 8% |
| cg07808555 | 1 | 11121771 2 | 5.76E-25 | 1.78 (1.6-1.99) | 5.76E-25 | 1.78 (1.6-1.99) | 5.54E-01 | 0% |
| cg02011074 | 1 | 17181046 8 | 7.17E-22 | 1.71 (1.53-1.91) | 2.12E-05 | 1.83 (1.38-2.42) | 2.96E-03 | 83% |
| cg26261793 | 1 | 17181054 3 | 1.26E-35 | 2.01 (1.8-2.24) | 5.96E-18 | 2.04 (1.73-2.39) | 1.38E-01 | 49% |
| cg06211893 | 1 | 17181077 8 | 2.19E-32 | 1.94 (1.74-2.16) | 1.26E-06 | 2.04 (1.53-2.73) | 1.90E-03 | 84% |
| cg05377226 | 1 | 17181091 0 | 1.82E-37 | 2.04 (1.83-2.28) | 2.90E-14 | 2.09 (1.73-2.53) | 6.49E-02 | 63% |
| cg23391785 | 1 | 17181097 2 | 5.87E-51 | 2.31 (2.07-2.58) | 7.19E-20 | 2.39 (1.98-2.88) | 7.12E-02 | 62% |
| cg26839871 | 1 | 17181129 9 | 1.52E-44 | 2.19 (1.96-2.44) | 2.96E-21 | 2.23 (1.89-2.64) | 1.22E-01 | 52% |
| cg10641721 | 3 | 13866500 6 | 1.69E-04 | 1.23 (1.11-1.38) | 1.00E-01 | 1.35 (0.94-1.93) | 6.76E-05 | 90% |
| cg05386493 | 3 | 13866565 4 | 2.13E-38 | 2.06 (1.85-2.3) | 1.65E-10 | 2.15 (1.7-2.71) | 1.62E-02 | 76% |
| cg18734304 | 3 | 13866581 6 | 8.44E-24 | 1.75 (1.57-1.96) | 5.67E-10 | 1.79 (1.49-2.16) | 7.64E-02 | 61% |
| cg13894046 | 3 | 13866593 0 | 4.62E-09 | 1.39 (1.24-1.55) | 1.35E-02 | 1.5 (1.09-2.08) | 3.68E-04 | 87% |
| cg25670330 | 3 | 13866598 4 | 4.31E-56 | 2.41 (2.16-2.69) | 1.78E-14 | 2.49 (1.97-3.14) | 1.71E-02 | 75% |
| cg15720669 | 3 | 13866604 0 | 1.68E-36 | 2.02 (1.81-2.26) | 1.32E-08 | 2.1 (1.62-2.71) | 7.42E-03 | 80% |
| cg04111064 | 3 | 13866604 8 | 2.60E-32 | 1.94 (1.74-2.16) | 1.69E-07 | 2.03 (1.56-2.64) | 5.10E-03 | 81% |
| cg17802942 | 3 | 13866605 0 | 1.03E-34 | 1.99 (1.78-2.22) | 1.24E-08 | 2.05 (1.6-2.62) | 1.02E-02 | 78% |
| cg17503456 | 3 | 13866605 4 | 3.60E-35 | 2 (1.79-2.23) | 4.11E-07 | 2.11 (1.58-2.82) | 1.81E-03 | 84% |

(continued)

| Marker | Chr | Position | Overall *P*-Value | Overall, OR (9% CI) | RE meta-analysis, *P*-Value | RE meta-analysis, OR (95% CI) | Cochran's *Q, P*-Value | *I²* |
|---|---|---|---|---|---|---|---|---|
| cg06186698 | 3 | 13866611 8 | 1.38E-30 | 1.9 (1.7-2.12) | 4.44E-15 | 1.95 (1.65-2.31) | 1.20E-01 | 53% |
| cg01379240 | 3 | 13866614 4 | 8.10E-53 | 2.35 (2.11-2.62) | 1.01E-21 | 2.42 (2.02-2.9) | 8.49E-02 | 59% |
| cg13740698 | 4 | 42399384 | 3.25E-18 | 1.63 (1.46-1.81) | 4.60E-15 | 1.63 (1.45-1.85) | 2.99E-01 | 17% |
| cg24084504 | 4 | 42399699 | 7.25E-30 | 1.89 (1.69-2.1) | 1.91E-13 | 1.94 (1.63-2.32) | 9.45E-02 | 58% |
| cg06269673 | 4 | 42399792 | 5.65E-24 | 1.76 (1.58-1.96) | 3.58E-10 | 1.8 (1.5-2.16) | 8.00E-02 | 60% |
| cg16862295 | 4 | 42399798 | 6.98E-33 | 1.95 (1.75-2.17) | 1.75E-17 | 1.98 (1.69-2.32) | 1.51E-01 | 47% |
| cg14866200 | 4 | 42399843 | 1.09E-52 | 2.35 (2.1-2.62) | 1.08E-11 | 2.47 (1.9-3.21) | 6.00E-03 | 80% |
| cg01565320 | 4 | 42399851 | 2.98E-55 | 2.4 (2.15-2.68) | 2.85E-13 | 2.51 (1.96-3.21) | 1.02E-02 | 78% |
| cg23684249 | 4 | 42399858 | 1.06E-34 | 1.99 (1.78-2.22) | 1.21E-09 | 2.09 (1.65-2.65) | 1.45E-02 | 76% |
| cg16094442 | 4 | 42400116 | 1.06E-15 | 1.57 (1.4-1.75) | 6.41E-07 | 1.6 (1.33-1.92) | 7.58E-02 | 61% |
| cg07638479 | 4 | 42400464 | 2.84E-18 | 1.63 (1.46-1.82) | 4.06E-05 | 1.72 (1.33-2.23) | 6.42E-03 | 80% |
| cg23841066 | 6 | 26250491 | 4.90E-07 | 1.32 (1.19-1.48) | 1.77E-02 | 1.42 (1.06-1.89) | 1.92E-03 | 84% |
| cg15386964 | 6 | 26250685 | 4.10E-55 | 2.4 (2.15-2.67) | 2.48E-35 | 2.42 (2.11-2.78) | 2.21E-01 | 34% |
| cg02578368 | 6 | 26250744 | 1.26E-33 | 1.96 (1.76-2.19) | 2.87E-05 | 2.15 (1.5-3.08) | 6.21E-05 | 90% |
| cg14808890 | 6 | 26250826 | 9.84E-24 | 1.75 (1.57-1.96) | 1.05E-04 | 1.9 (1.37-2.62) | 3.77E-04 | 87% |
| cg14159026 | 6 | 10558455 1 | 1.15E-61 | 2.52 (2.26-2.82) | 2.41E-19 | 2.57 (2.09-3.15) | 4.17E-02 | 69% |
| cg10979880 | 6 | 10558468 9 | 5.81E-32 | 1.93 (1.73-2.15) | 6.28E-10 | 2 (1.61-2.49) | 2.63E-02 | 73% |
| cg14314653 | 6 | 10558470 9 | 1.65E-40 | 2.1 (1.89-2.35) | 2.85E-10 | 2.2 (1.72-2.81) | 1.10E-02 | 78% |
| cg13725782 | 6 | 10558471 8 | 1.57E-40 | 2.11 (1.89-2.35) | 9.14E-18 | 2.15 (1.8-2.56) | 9.97E-02 | 57% |
| cg01513081 | 6 | 10558478 0 | 4.60E-25 | 1.78 (1.6-1.99) | 6.07E-15 | 1.79 (1.55-2.08) | 1.92E-01 | 39% |
| cg26449787 | 6 | 13356208 7 | 1.41E-30 | 1.9 (1.7-2.12) | 4.55E-13 | 1.93 (1.61-2.3) | 9.21E-02 | 58% |

(continued)

| Marker | Chr | Position | Overall *P*-Value | Overall, OR (9% CI) | RE meta-analysis, *P*-Value | RE meta-analysis, OR (95% CI) | Cochran's *Q, P*-Value | *I²* |
|---|---|---|---|---|---|---|---|---|
| cg14017655 | 6 | 13356219 3 | 1.53E-10 | 1.43 (1.28-1.6) | 7.70E-04 | 1.5 (1.18-1.9) | 1.51E-02 | 76% |
| cg06181518 | 6 | 13356219 6 | 8.14E-11 | 1.44 (1.29-1.6) | 4.88E-06 | 1.47 (1.25-1.74) | 1.24E-01 | 52% |
| cg11518846 | 6 | 13356224 6 | 2.21E-21 | 1.7 (1.52-1.9) | 1.17E-05 | 1.78 (1.37-2.3) | 7.00E-03 | 80% |
| cg20980055 | 6 | 13356225 8 | 1.12E-24 | 1.77 (1.59-1.98) | 5.41E-07 | 1.81 (1.44-2.29) | 1.71E-02 | 75% |
| cg20286200 | 6 | 13356226 7 | 1.79E-16 | 1.58 (1.42-1.77) | 4.66E-04 | 1.66 (1.25-2.2) | 2.50E-03 | 83% |
| cg05062333 | 6 | 13356226 9 | 1.11E-21 | 1.71 (1.53-1.9) | 1.51E-05 | 1.79 (1.38-2.33) | 5.27E-03 | 81% |
| cg01162672 | 6 | 13356227 5 | 5.10E-11 | 1.44 (1.29-1.61) | 6.92E-04 | 1.51 (1.19-1.92) | 1.34E-02 | 77% |
| cg01328892 | 6 | 13356233 2 | 2.34E-04 | 1.23 (1.1-1.37) | 6.90E-02 | 1.31 (0.98-1.75) | 1.81E-03 | 84% |
| cg05085230 | 6 | 13356246 1 | 4.93E-30 | 1.89 (1.69-2.11) | 1.36E-11 | 1.91 (1.58-2.31) | 7.03E-02 | 62% |
| cg08712932 | 6 | 13356246 3 | 1.89E-33 | 1.96 (1.76-2.19) | 1.36E-17 | 1.96 (1.68-2.29) | 1.60E-01 | 45% |
| cg06132028 | 6 | 13356246 6 | 8.00E-35 | 1.99 (1.78-2.22) | 2.01E-18 | 2.02 (1.72-2.36) | 1.52E-01 | 47% |
| cg11942956 | 6 | 13356247 0 | 1.38E-36 | 2.03 (1.82-2.26) | 5.12E-27 | 2.03 (1.78-2.31) | 2.71E-01 | 23% |
| cg11664500 | 6 | 13356247 9 | 1.08E-45 | 2.21 (1.98-2.46) | 2.51E-18 | 2.25 (1.88-2.7) | 8.17E-02 | 60% |
| cg14287112 | 6 | 13356248 5 | 1.07E-48 | 2.27 (2.03-2.53) | 1.37E-15 | 2.33 (1.89-2.87) | 3.91E-02 | 69% |
| cg22871668 | 6 | 13356249 2 | 2.03E-50 | 2.3 (2.06-2.57) | 1.99E-12 | 2.39 (1.88-3.05) | 1.18E-02 | 77% |
| cg14270292 | 6 | 13356249 4 | 9.96E-37 | 2.03 (1.82-2.26) | 6.01E-11 | 2.1 (1.68-2.62) | 2.46E-02 | 73% |
| cg26656135 | 6 | 13356277 4 | 8.92E-20 | 1.66 (1.49-1.85) | 2.23E-04 | 1.72 (1.29-2.3) | 1.95E-03 | 84% |
| cg24176563 | 6 | 13356277 6 | 7.01E-14 | 1.52 (1.36-1.7) | 1.40E-03 | 1.58 (1.19-2.1) | 2.54E-03 | 83% |
| cg01401376 | 6 | 13356334 2 | 1.53E-13 | 1.51 (1.35-1.69) | 3.60E-05 | 1.53 (1.25-1.86) | 4.87E-02 | 67% |
| cg21607030 | 6 | 13356353 2 | 1.54E-34 | 1.98 (1.78-2.21) | 8.95E-08 | 2.06 (1.58-2.68) | 5.23E-03 | 81% |
| cg10297491 | 10 | 11059677 | 8.82E-37 | 2.03 (1.82-2.26) | 1.67E-33 | 2.03 (1.81-2.28) | 3.38E-01 | 8% |

(continued)

| Marker | Chr | Position | Overall *P*-Value | Overall, OR (9% CI) | RE meta-analysis, *P*-Value | RE meta-analysis, OR (95% CI) | Cochran's *Q, P*-Value | *I²* |
|---|---|---|---|---|---|---|---|---|
| cg27041794 | 10 | 11059708 | 6.96E-32 | 1.93 (1.73-2.15) | 6.99E-10 | 2.01 (1.61-2.51) | 2.50E-02 | 73% |
| cg17290701 | 10 | 11059714 | 1.15E-43 | 2.17 (1.94-2.42) | 1.48E-26 | 2.19 (1.9-2.53) | 2.02E-01 | 37% |
| cg26328510 | 10 | 11059718 | 1.93E-51 | 2.32 (2.08-2.59) | 2.70E-19 | 2.38 (1.97-2.88) | 6.69E-02 | 63% |
| cg03813164 | 10 | 11059725 | 4.77E-53 | 2.35 (2.11-2.63) | 1.71E-16 | 2.44 (1.97-3.01) | 3.44E-02 | 70% |
| cg12356890 | 10 | 11059727 | 6.62E-59 | 2.47 (2.21-2.75) | 7.77E-34 | 2.5 (2.15-2.9) | 1.86E-01 | 40% |
| cg11472279 | 10 | 11059733 | 1.35E-53 | 2.37 (2.12-2.64) | 8.25E-17 | 2.45 (1.98-3.03) | 3.52E-02 | 70% |
| cg11002404 | 10 | 11060038 | 3.39E-48 | 2.26 (2.02-2.52) | 3.39E-48 | 2.26 (2.02-2.52) | 5.39E-01 | 0% |
| cg04088969 | 10 | 11060048 | 3.40E-34 | 1.98 (1.77-2.2) | 1.82E-08 | 2.1 (1.62-2.71) | 6.81E-03 | 80% |
| cg15556502 | 11 | 43602845 | 6.12E-38 | 2.05 (1.84-2.29) | 1.87E-14 | 2.09 (1.73-2.53) | 6.78E-02 | 63% |
| cg14416371 | 11 | 43602847 | 2.35E-51 | 2.32 (2.08-2.59) | 3.61E-13 | 2.41 (1.9-3.05) | 1.48E-02 | 76% |
| cg14944647 | 11 | 43602857 | 2.22E-38 | 2.06 (1.85-2.3) | 1.04E-18 | 2.09 (1.77-2.45) | 1.33E-01 | 51% |
| cg01939477 | 11 | 43602879 | 1.46E-49 | 2.29 (2.05-2.55) | 3.21E-19 | 2.32 (1.93-2.79) | 7.77E-02 | 61% |
| cg16407471 | 11 | 43602914 | 2.82E-35 | 2 (1.79-2.23) | 1.52E-11 | 2.05 (1.67-2.53) | 3.73E-02 | 70% |
| cg05376374 | 11 | 43602920 | 7.34E-41 | 2.11 (1.89-2.36) | 1.21E-15 | 2.16 (1.79-2.61) | 6.81E-02 | 63% |
| cg03365311 | 11 | 43602965 | 2.43E-39 | 2.08 (1.87-2.32) | 1.29E-13 | 2.14 (1.75-2.62) | 4.70E-02 | 67% |
| cg04279973 | 16 | 23846968 | 2.74E-33 | 1.96 (1.75-2.18) | 3.51E-06 | 2.11 (1.54-2.89) | 5.74E-04 | 87% |
| cg03306374 | 16 | 23847325 | 3.15E-38 | 2.06 (1.85-2.3) | 4.84E-14 | 2.13 (1.75-2.59) | 5.54E-02 | 65% |
| cg03217795 | 16 | 23847556 | 8.34E-52 | 2.33 (2.09-2.6) | 1.75E-23 | 2.38 (2.01-2.82) | 1.12E-01 | 54% |
| cg05436658 | 16 | 23847568 | 8.39E-39 | 2.07 (1.86-2.31) | 9.01E-26 | 2.07 (1.81-2.37) | 2.37E-01 | 31% |
| cg03156893 | 16 | 23847675 | 1.47E-09 | 1.4 (1.26-1.56) | 3.76E-02 | 1.57 (1.03-2.39) | 1.32E-06 | 93% |
| cg09507526 | 16 | 23847816 | 1.31E-22 | 1.73 (1.55-1.93) | 8.60E-08 | 1.81 (1.46-2.24) | 2.93E-02 | 72% |

(continued)

| Marker | Chr | Position | Overall P-Value | Overall, OR (9% CI) | RE meta-analysis, P-Value | RE meta-analysis, OR (95% CI) | Cochran's Q, P-Value | $I^2$ |
|---|---|---|---|---|---|---|---|---|
| cg21370856 | 16 | 23848003 | 9.41E-33 | 1.95 (1.74-2.17) | 4.75E-08 | 2.02 (1.57-2.61) | 8.18E-03 | 79% |
| cg19554255 | 17 | 75369051 | 4.81E-40 | 2.1 (1.88-2.34) | 4.81E-40 | 2.1 (1.88-2.34) | 4.34E-01 | 0% |
| cg16779463 | 17 | 75369055 | 3.72E-38 | 2.06 (1.84-2.3) | 3.72E-38 | 2.06 (1.84-2.3) | 5.41E-01 | 0% |
| cg17300544 | 17 | 75369091 | 3.53E-50 | 2.3 (2.06-2.56) | 7.35E-37 | 2.31 (2.03-2.63) | 2.67E-01 | 24% |
| cg03804136 | 17 | 75369219 | 1.61E-44 | 2.19 (1.96-2.44) | 1.77E-39 | 2.19 (1.95-2.46) | 3.30E-01 | 10% |
| cg15044248 | 17 | 75369224 | 5.74E-45 | 2.19 (1.97-2.45) | 4.53E-42 | 2.2 (1.96-2.46) | 3.47E-01 | 5% |
| cg02884239 | 17 | 75369228 | 1.05E-33 | 1.97 (1.76-2.19) | 1.05E-33 | 1.97 (1.76-2.19) | 6.97E-01 | 0% |
| cg20275528 | 17 | 75369484 | 1.02E-46 | 2.23 (2-2.49) | 1.02E-46 | 2.23 (2-2.49) | 4.80E-01 | 0% |
| cg12783819 | 17 | 75369657 | 2.61E-47 | 2.24 (2.01-2.5) | 2.61E-47 | 2.24 (2.01-2.5) | 5.90E-01 | 0% |
| cg05184938 | 17 | 75369939 | 1.15E-37 | 2.05 (1.83-2.28) | 1.15E-37 | 2.05 (1.83-2.28) | 4.63E-01 | 0% |
| cg16692998 | 19 | 19739173 | 5.00E-44 | 2.18 (1.95-2.43) | 2.00E-25 | 2.21 (1.9-2.56) | 1.83E-01 | 41% |
| cg17419241 | 19 | 19739180 | 1.09E-29 | 1.88 (1.69-2.1) | 7.58E-16 | 1.91 (1.63-2.24) | 1.50E-01 | 47% |
| cg02362385 | 19 | 19739192 | 4.17E-37 | 2.04 (1.82-2.27) | 1.78E-24 | 2.06 (1.8-2.37) | 2.23E-01 | 33% |
| cg04395369 | 19 | 19739209 | 2.89E-31 | 1.91 (1.72-2.14) | 5.36E-13 | 1.94 (1.62-2.32) | 8.69E-02 | 59% |
| cg19306047 | 19 | 19739407 | 5.71E-52 | 2.33 (2.09-2.6) | 9.67E-32 | 2.36 (2.04-2.72) | 2.05E-01 | 37% |

The HCC Epigenome Score had AUROCs of 0.892 (95% CI, 0.804-0.980; P < 0.0001) and 0.928 (95% CI, 0.902-0.954; P < 0.0001), respectively. In the random-effect meta-analysis that pooled the initial and the two replication studies, the AUROC of the HCC Epigenome Score was 0.928 (95% CI, 0.904-0.953; $P$ < 0.001; $I^2$ = 0% (95% CI, 0-95%). Using a combined dataset from all the studied samples (586, 353 HCC, 233 No HCC), the HCC Epienome score significantly differed between HCC (median, 0.34 [IQR, 0.238-0.464]) and non-HCC samples (median, 0.10 [IQR, 0.08-0.12]) (P < 0.0001) **(Figure 6A).** In ROC analysis, the HCC Epigenome score had an AUROC of 0.955 (95% CI, 0.935 to 0.969; $P$ < 0.0001) to detect HCC (See, Supplementary Appendix). The inventors performed dose-response probit regression analysis to assess the association between the HCC Epigenome Score using the 586 samples (353 HCC, 233 No HCC). The HCC Epigenome score was significantly associated with the risk of HCC with a gradual increase in the risk of HCC according to the increase in the HCC Epigenome Score. The Nagelkerke $R^2$ of 0.7735 (P < 0.0001) **(Table 3, Figure 6B).**

| Probability | Dose | 95% CI | |
|:---:|:---:|:---:|:---:|
| 0.01 | 0.030 | -0.003 | 0.052 |
| 0.02 | 0.045 | 0.017 | 0.065 |
| 0.025 | 0.051 | 0.023 | 0.069 |
| 0.05 | 0.068 | 0.045 | 0.084 |
| 0.1 | 0.088 | 0.070 | 0.102 |
| 0.2 | 0.113 | 0.099 | 0.125 |
| 0.25 | 0.122 | 0.109 | 0.134 |
| 0.5 | 0.160 | 0.148 | 0.174 |
| 0.75 | 0.197 | 0.182 | 0.219 |
| 0.8 | 0.206 | 0.190 | 0.231 |
| 0.9 | 0.231 | 0.210 | 0.261 |
| 0.95 | 0.251 | 0.227 | 0.287 |
| 0.975 | 0.268 | 0.242 | 0.309 |
| 0.98 | 0.274 | 0.246 | 0.316 |
| 0.99 | 0.289 | 0.259 | 0.335 |

**Table 3. Association between the HCC Epigenome score and the risk of HCC in dose-response probit regression analysis.**

## Claims

1. A method for diagnosing hepatocellular carcinoma (HCC) in a subject comprising the step of determining the methylation level of at least one CpG site within each of CpG islands listed in Table 1 in DNA sample obtained from said subject, wherein a higher methylation level of said CpG sites as compared to a control value is indicative that the subject has a hepatocellular carcinoma.

2. The method of claim 1 wherein said CpG sites are selected from the group consisting of CpG sites listed in Table 1.

3. The method according to claim 1 or 2 wherein said methylation level is the ratio of methylated cytosine and the sum of methylated and unmethylated cytosines.

4. The method according to any of the preceding claims wherein the step of determining said methylation level comprises a bisulfite pre-treatment of the DNA samples which converts unmethylated cytosines into uracils.

5. The method according to any of the preceding claims wherein the step of determining said methylation level comprises applying DNA samples on microarray chips of probes.

6. The method according to any one of claims 1 to 5 wherein the control value corresponds to methylation level of said CpG sites in a biological sample obtained from a subject who have not hepatocellular carcinoma.

7. The method according to any of the preceding claims wherein said biological sample is circulating free DNA.

8. A kit for predicting hepatocellular carcinoma disease in a subject which comprises a set of probes which each hybridizes with a nucleic acid sequence comprising at least one CpG site within each CpG islands listed in Table 1.

9. The kit of claim 8 wherein said CpG sites are selected from the group consisting of CpG sites listed in Table 1.

10. The kit of claim 8 or 9 further comprising a set of probes which each hybridizes with a nucleic acid sequence comprising at least one CpG site within each CpG islands listed in Table 1, wherein cytosines are replaced by uracils.

**11.** The kit of claim 10 wherein said CpG sites are selected from the group consisting of CpG sites listed in Table 1.


**Patentansprüche**

**1.** Verfahren zum Diagnostizieren von hepatozellulärem Karzinom (HCC) in einem Individuum, umfassend den Schritt des Bestimmens des Methylierungsgrades von mindestens einer CpG-Stelle innerhalb jeder der in Tabelle 1 aufgeführten CpG-Inseln in einer von dem Individuum erhaltenen DNA-Probe, wobei ein höherer Methylierungsgrad der CpG-Stellen im Vergleich mit einem Kontrollwert anzeigt, dass das Individuum ein hepatozelluläres Karzinom aufweist.

**2.** Verfahren nach Anspruch 1, wobei die CpG-Stellen ausgewählt sind aus der Gruppe bestehend aus den in Tabelle 1 aufgeführten CpG-Stellen.

**3.** Verfahren nach Anspruch 1 oder 2, wobei der Methylierungsgrad das Verhältnis von methyliertem Cytosin und der Summe von methylierten und unmethylierten Cytosinen ist.

**4.** Verfahren nach einem der vorherstehenden Ansprüche, wobei der Schritt des Bestimmens des Methylierungsgrades eine Bisulfit-Vorbehandlung der DNA-Proben umfasst, welche unmethylierte Cytosine zu Uracilen konvertiert.

**5.** Verfahren nach einem der vorherstehenden Ansprüche, wobei der Schritt des Bestimmens des Methylierungsgrades Aufbringen von DNA-Proben auf Microarray-Chips von Sonden umfasst.

**6.** Verfahren nach einem der Ansprüche 1 bis 5, wobei der Kontrollwert einem Methylierungsgrad der CpG-Stellen in einer biologischen Probe entspricht, welche von einem Individuum erhalten wurde, welches kein hepatozelluläres Karzinom aufweist.

**7.** Verfahren nach einem der vorherstehenden Ansprüche, wobei die biologische Probe zirkulierende freie DNA ist.

**8.** Kit zum Vorhersagen einer Erkrankung an hepatozellulärem Karzinom in einem Individuum, welches einen Satz von Sonden umfasst, welche jeweils mit einer Nukleinsäuresequenz hybridisieren, welche mindestens eine CpG-Stelle innerhalb jeder der in Tabelle 1 aufgeführten CpG-Inseln umfasst.

**9.** Kit nach Anspruch 8, wobei die CpG-Stellen ausgewählt sind aus der Gruppe bestehend aus den in Tabelle 1 aufgeführten CpG-Stellen.

**10.** Kit nach Anspruch 8 oder 9, ferner umfassend einen Satz von Sonden, welche jeweils mit einer Nukleinsäuresequenz hybridisieren, welche mindestens eine CpG-Stelle innerhalb jeder der in Tabelle 1 aufgeführten CpG-Inseln umfasst, wobei Cytosine durch Uracile ersetzt sind.

**11.** Kit nach Anspruch 10, wobei die CpG-Stellen ausgewählt sind aus der Gruppe bestehend aus den in Tabelle 1 aufgeführten CpG-Stellen.


**Revendications**

**1.** Procédé de diagnostic du carcinome hépatocellulaire (HCC) chez un sujet comprenant l'étape de la détermination du niveau de méthylation d'au moins un site de CpG au sein de chacun des îlots de CpG listés dans le Tableau 1 dans un échantillon d'ADN obtenu auprès dudit sujet, dans lequel un niveau de méthylation plus élevé desdits sites de CpG comparativement à une valeur de contrôle est indicatif du fait que le sujet présente un carcinome hépatocellulaire.

**2.** Procédé selon la revendication 1 dans lequel lesdits sites de CpG sont sélectionnés parmi le groupe consistant en les sites de CpG listés dans le Tableau 1.

**3.** Procédé selon la revendication 1 ou 2 dans lequel ledit niveau de méthylation est le rapport de la cytosine méthylée et de la somme des cytosines méthylées et non méthylées.

**4.** Procédé selon l'une quelconque des revendications précédentes dans lequel l'étape de la détermination dudit niveau de méthylation comprend un prétraitement au bisulfite des échantillons d'ADN qui convertit les cytosines non méthylées en uraciles.

5. Procédé selon l'une quelconque des revendications précédentes dans lequel l'étape de la détermination dudit niveau de méthylation comprend l'application d'échantillons d'ADN sur des puces de sondes en microréseau.

6. Procédé selon l'une quelconque des revendications 1 à 5 dans lequel la valeur de contrôle correspond au niveau de méthylation desdits sites de CpG dans un échantillon biologique obtenu auprès d'un sujet que ne présente pas de carcinome hépatocellulaire.

7. Procédé selon l'une quelconque des revendications précédentes dans lequel ledit échantillon biologique est de l'ADN libre circulant.

8. Kit pour la prédiction du carcinome hépatocellulaire chez un sujet qui comprend un ensemble de sondes qui s'hybrident chacune avec une séquence d'acides nucléiques comprenant au moins un site de CpG au sein de chacun des îlots de CpG listés dans le Tableau 1.

9. Kit selon la revendication 8 dans lequel lesdits sites de CpG sont sélectionnés parmi le groupe consistant en les sites de CpG listés dans le Tableau 1.

10. Kit selon la revendication 8 ou 9 comprenant en outre un ensemble de sondes qui s'hybrident chacune avec une séquence d'acides nucléiques comprenant au moins un site de CpG au sein de chacun des îlots de CpG listés dans le Tableau 1, dans lequel les cytosines sont remplacées par des uraciles.

11. Kit selon la revendication 10 dans lequel lesdits sites de CpG sont sélectionnés parmi le groupe consistant en les sites de CpG listés dans le Tableau 1.

# No smoothing (radius = 0)

Unsmoothed -Log10(P-value)

# Smoothing radius = $r$

$$k\text{-}3 \quad k\text{-}2 \quad k\text{-}1 \quad k \quad k\text{+}1 \quad k\text{+}2 \quad k\text{+}3$$

$$\mathbf{Smoothed}(Pk, w) = \sum_{n=k-w}^{n=k+w} (-Log10[P - value]) \div (2w + 1)$$

FIG. 1

EP 4 419 717 B1

**FIG. 2**

Initial study, smoothed method (w = 3)

(1) RUNX3　　　　　(8) EYA4
(2) KCNA3　　　　　(9) CELF2
(3) DNM3　　　　　 (10) MIR129-2
(4) FOXL2/FOXL2NB　(11) PRKCB
(5) SHISA3　　　　 (12) SEPT9
(6) H3C7　　　　　 (13) LPAR2
(7) BVES

FIG. 3

FIG. 4

**FIG. 5**

A — Initial study, smoothed method ($w = 3$)

B — Replication study #1

−Log10(P-value)

(1) RUNX3
(2) KCNA3
(3) DNM3
(4) FOXL2/FOXL2NB
(5) SHISA3
(6) H3C7
(7) BVES
(8) EYA4
(9) CELF2
(10) MIR129-2
(11) PRKCB
(12) SEPT9
(13) LPAR2

**C** Replication study #2

**D** Random-effect meta-analysis (initial, replication #1, and replication #2)

(1) *RUNX3*
(2) *KCNA3*
(3) *DNM3*
(4) *FOXL2/FOXL2NB*
(5) *SHISA3*
(6) *H3C7*
(7) *BVES*
(8) *EYA4*
(9) *CELF2*
(10) *MIR129-2*
(11) *PRKCB*
(12) *SEPT9*
(13) *LPAR2*

**FIG. 5**

**FIG. 6**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2021146570 A
- WO 02086163 A1
- US 5786146 A
- US 6265171 B
- US 6251594 B
- US 6331393 B
- US 7611869 B
- WO 2004051224 A

## Non-patent literature cited in the description

- **EL-SERAG HB**. Hepatocellular carcinoma. *N Engl J Med*, 2011, vol. 365, 1118-1127
- **BRUIX J** ; **REIG M** ; **SHERMAN M**. *Gastroenterology*, 2016, vol. 150, 835-853
- **OUSSALAH A et al.** *Oncotarget*, 2016
- *Cancer Fact sheet*, 15 December 2017, http://www.who.int/mediacentre/factsheets/fs297/en
- **MORAN S et al.** *Nat Rev Clin Oncol*, 2017
- **HAO X et al.** *Proc Natl Acad Sci U S A*, 2017, vol. 114, 7414-7419
- **MICHELS KB et al.** *Nat Methods*, 2013, vol. 10, 949-955
- **VAN ITERSON M** ; **VAN ZWET EW** ; **CONSORTIUM B** ; **HEIJMANS BT**. *Genome Biol*, 2017, vol. 18, 19
- **HUANG et al.** *Human Mol. Genet.*, 1999, vol. 8, 459-70
- **PLASS et al.** *Genomics*, 1999, vol. 58, 254-62
- **GONZALGO et al.** *Cancer Res.*, 1997, vol. 57, 594-599
- **TOYOTA et al.** *Cancer Res.*, 1999, vol. 59, 2307-2312
- **CLARK et al.** *Nucleic Acids Res.*, 1994, vol. 22, 2990-2997
- **FROMMER et al.** *PNAS USA*, 1992, vol. 89, 1827-183 1
- **HERMAN et al.** *Proc. Natl. Acad. Sci. USA*, 1996, vol. 93, 9821-9826
- **GONZALGO** ; **JONES**. *Nucleic Acids Res.*, 1997, vol. 25, 2529-253 1
- **EADS et al.** *Cancer Res.*, 1999, vol. 59, 2302-2306
- **HEID et al.** *Genome Res.*, 1996, vol. 6, 986-994
- **CONSORTIUM EP**. *Nature*, 2012, vol. 489, 57-74
- **DALENIUS T** ; **HODGES JR JL**. *J Am Stat Assoc*, 1959, vol. 54, 88-101
- **ECKHARDT, F. et al.** *Nat Genet*, 2006, vol. 38, 1378-1385
- **BIBIKOVA, M. et al.** *Genomics*, 2011, vol. 98, 288-295
- **DELONG ER et al.** *Biometrics*, 1988, vol. 44, 837-845
- **EFRON B** ; **TIBSHIRANI RJ**. An Introduction to the Bootstrap. Taylor & Francis, 1994
- **HIGGINS JP et al.** *BMJ*, 2003, vol. 327, 557-560